(19) Europäisches Patentamt
European Patent Office
Office européen des brevets

(11) **EP 4 455 645 A1**

(12) **EUROPEAN PATENT APPLICATION**
published in accordance with Art. 153(4) EPC

(43) Date of publication:
**30.10.2024 Bulletin 2024/44**

(21) Application number: **22910827.9**

(22) Date of filing: **02.12.2022**

(51) International Patent Classification (IPC):
*G01N 21/88* (2006.01)     *B23K 31/00* (2006.01)
*G01B 11/24* (2006.01)     *G06N 20/00* (2019.01)

(52) Cooperative Patent Classification (CPC):
**B23K 31/00; G01B 11/24; G01N 21/88; G06N 20/00**

(86) International application number:
**PCT/JP2022/044531**

(87) International publication number:
**WO 2023/120110 (29.06.2023 Gazette 2023/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.12.2021 JP 2021210112**

(71) Applicant: **Panasonic Intellectual Property Management Co., Ltd.**
**Osaka-shi, Osaka 540-6207 (JP)**

(72) Inventors:
• **SAKAI Toru**
**Kadoma-shi, Osaka 571-0057 (JP)**
• **YOSHIDA Masashi**
**Kadoma-shi, Osaka 571-0057 (JP)**
• **SAKURAI Michio**
**Kadoma-shi, Osaka 571-0057 (JP)**
• **HIGASHI Daichi**
**Kadoma-shi, Osaka 571-0057 (JP)**

(74) Representative: **Novagraaf International SA**
**Chemin de l'Echo 3**
**1213 Onex, Geneva (CH)**

(54) **APPEARANCE INSPECTING DEVICE, WELDING SYSTEM, SHAPE DATA CORRECTING METHOD, AND WELDING LOCATION APPEARANCE INSPECTING METHOD**

(57)     An appearance inspection apparatus 20 includes a shape measurement unit 21 configured to measure the three-dimensional shape of a weld 201 and a data processor 23 configured to process shape data acquired by the shape measurement unit 21. The data processor 23 includes a shape data processor 24 configured to correct a resolution of the shape data, a learning data set generator 26 configured to generate a plurality of learning data sets by performing data augmentation on multiple pieces of sample shape data acquired in advance, a determination model generator 27 configured to generate a determination model using the plurality of learning data sets, and a first determination unit 28 configured to determine whether the shape of the weld 201 is good or bad based on the shape data having the corrected resolution and the determination model.

FIG.3

**Description**

TECHNICAL FIELD

The present disclosure relates to an appearance inspection apparatus, a welding system, a method for correcting shape data, and a method for appearance inspection of a weld.

BACKGROUND ART

Inspecting the appearance of a weld using a determination model reinforced by machine learning to determine whether the shape of the weld is good or bad has recently become popular.

For example, Patent Document 1 proposes a weld appearance inspection apparatus including a shape measurement unit, an image processor, a learning data set generator, a determination model generator, and a first determination unit.

The shape measurement unit measures the shape of the weld, and the image processor generates image data of the weld based on shape data measured. The learning data set generator classifies multiple pieces of image data by material and shape of a workpiece and performs data augmentation to generate a plurality of learning data sets. The determination model generator generates a determination model for the shape of the weld for each material and shape of the workpiece using the plurality of learning data sets. The first determination unit determines whether the shape of the weld is good or bad based on the image data read from the image processor and the determination model.

CITATION LIST

PATENT DOCUMENT

Patent Document 1: International Patent Publication No. WO 2020/129617

SUMMARY OF THE INVENTION

TECHNICAL PROBLEM

In an actual production processing site of workpieces, inspection conditions of the appearance inspection apparatus are changed as appropriate. For example, conditions such as an inspection speed for inspecting the weld along a welding line and a measurement frequency and measurement resolution of a sensor are successively changed to be optimum to the purpose of the user of the processing facility.

The inspection conditions are changed in this way because production takt time for the workpiece and inspection accuracy greatly vary depending on the inspection conditions. For example, when the production takt time is important, the inspection speed is set higher to perform the inspection in a shorter production takt time. However, this lowers the measurement resolution of the sensor, making the three-dimensional shape of the weld acquired by the sensor coarse. Thus, small weld defects cannot be detected, lowering the inspection accuracy.

When the inspection accuracy is important, the inspection speed is lowered to increase the measurement resolution. However, the lowered inspection speed increases the production takt time.

The inspection conditions are also changed depending on the status of the workpiece to be inspected or the weld. For example, the inspection speed may be changed depending on whether the shape of the workpiece is curved or linear. A product that does not allow any small defects requires more accurate inspection. In this case, the measurement resolution is increased at the cost of the production takt time.

Due to wide varieties of materials of the workpiece to be inspected and shapes of the welds, the inspection conditions are adjusted to the shapes of the welds.

However, for example, when the measurement resolution of the sensor changes, the measurement result differs even if the three-dimensional shape of the same weld is measured. Thus, the feature of the shape data of the weld inputted to the determination model does not match the feature of the shape data obtained in advance by machine learning, deteriorating the accuracy of the appearance inspection.

In view of the foregoing, an object of the present disclosure is to provide an appearance inspection apparatus, a welding system, a method for correcting shape data, and a method for appearance inspection of a weld that allows for accurate evaluation of the three-dimensional shape of the weld although inspection conditions are changed.

SOLUTION TO THE PROBLEM

To achieve the object, the present disclosure provides an appearance inspection apparatus for inspecting an appearance of a weld of a workpiece. The appearance inspection apparatus includes at least: a shape measurement unit that is attached to a robot and configured to measure a three-dimensional shape of the weld along a welding line; and a data processor configured to process shape data acquired by the shape measurement unit. The data processor includes at least a shape data processor configured to perform at least correction of a resolution of the shape data acquired by the shape measurement unit, a learning data set generator configured to generate a plurality of learning data sets by performing data augmentation on multiple pieces of sample shape data acquired in advance by the shape measurement unit, a determination model generator configured to generate a determination model for determining whether the shape of the weld is good or bad using the plurality of learning data sets, and a first determination unit configured to determine whether the shape of the weld is good or bad based on the shape data corrected by the shape data processor and one or more determination models generated by the determination model generator.

A welding system of the present disclosure includes the appearance inspection apparatus and a welding apparatus that welds the workpiece. The welding apparatus includes at least a welding head configured to apply heat to the workpiece and an output controller configured to control a welding output of the welding head.

A method for correcting shape data of the present disclosure is a method for correcting shape data acquired by the appearance inspection apparatus. The method includes:

measuring a three-dimensional shape of the weld by the shape measurement unit moving together with the robot to acquire the sample shape data for generating the plurality of learning data sets; measuring the three-dimensional shape of the weld by the shape measurement unit moving together with the robot to acquire the shape data; and correcting,
when a resolution of the shape data acquired by the shape measurement unit is different from a resolution of the sample shape data, the shape data by the shape data processor so that the resolution of the shape data acquired by the shape measurement unit has the same value as the resolution of the sample shape data.

A method for appearance inspection of a weld of the present disclosure is a method for appearance inspection of a weld using the appearance inspection apparatus. The method includes at least: measuring a three-dimensional shape of the weld by the shape measurement unit moving together with the robot to acquire the sample shape data for generating the plurality of learning data sets; generating one or more determination models for determining whether the shape of the weld is good or bad by the determination model generator using the plurality of learning data sets; measuring the three-dimensional shape of the weld by the shape measurement unit moving together with the robot to acquire the shape data; correcting, when a resolution of the shape data acquired by the shape measurement unit is different from a resolution of the sample shape data, the shape data by the shape data processor so that the resolution of the shape data acquired by the shape measurement unit has the same value as the resolution of the sample shape data; and determining whether the shape of the weld is good or bad by the first determination unit based on the shape data having the resolution corrected by the shape data processor and the one or more determination models generated by the determination model generator.

ADVANTAGES OF THE INVENTION

According to the present disclosure, a three-dimensional shape of a weld can be accurately evaluated although inspection conditions are changed. Further, whether the shape of the weld is good or bad can be correctly determined.

BRIEF DESCRIPTION OF THE DRAWINGS

FIG. 1 is a schematic view of a configuration of a welding system according to an embodiment.
FIG. 2 is a schematic view of a hardware configuration of a robot processor.
FIG. 3 is a functional block diagram of an appearance inspection apparatus.
FIG. 4 is a schematic view of measurement of the shape of a weld bead by a shape measurement unit.
FIG. 5A is a schematic view of a hardware configuration of a sensor controller.
FIG. 5B is a schematic view of a hardware configuration of a data processor.
FIG. 6A is a schematic plan view of an example of a defective mode of a weld.
FIG. 6B is a schematic cross-sectional view taken along line VIB-VIB of FIG. 6A.
FIG. 6C is a schematic cross-sectional view taken along line VIC-VIC of FIG. 6A.

FIG. 6D is a schematic cross-sectional view taken along line VID-VID of FIG. 6A.

FIG. 6E is a schematic cross-sectional view taken along line VIE-VIE of FIG. 6A.

FIG. 7A is a schematic view of an example of a procedure for generating a learning data set.

FIG. 7B is a schematic view of another example of the procedure for generating the learning data set.

FIG. 7C is a schematic view of another example of the procedure for generating the learning data set.

FIG. 8A is a flowchart of a procedure for weld appearance inspection.

FIG. 8B is a flowchart of a procedure for determining whether the shape of a weld bead is good or bad.

FIG. 9 is a conceptual diagram illustrating a procedure for deriving coordinate points of shape data in conversion/correction of a resolution.

FIG. 10 is a conceptual diagram illustrating how the positions of coordinate points in the shape data change before and after the conversion/correction of the resolution in an acceleration/deceleration section.

FIG. 11 is a schematic view of an example of a speed control function of a robot in the acceleration/deceleration section.

FIG. 12 is a schematic view of appearance inspection of a weld according to Example 1.

FIG. 13 is a schematic view of the planar shape of a weld according to Examples 2 and 3.

FIG. 14 is a schematic view of appearance inspection of a weld according to Example 4.

FIG. 15 is a schematic view of a Z-direction profile of a weld according to Example 5.

FIG. 16A is a schematic view of an arrangement of a shape measurement unit is arranged in section A.

FIG. 16B is a schematic view of an arrangement of the shape measurement unit in section B.

FIG. 16C is a schematic view of an arrangement of the shape measurement unit in section C.

FIG. 17A is a schematic view of an arrangement of the shape measurement unit by a conventional method for the inspection of section A.

FIG. 17B is a schematic view of an arrangement of the shape measurement unit by a conventional method for the inspection of section B.

DESCRIPTION OF EMBODIMENTS

Embodiments of the present disclosure will be described below with reference to the drawings. The following description of the embodiments is merely an example in nature, and is not intended to limit the scope, applications, or use of the present invention.

(Embodiments)

[Configuration of Welding System]

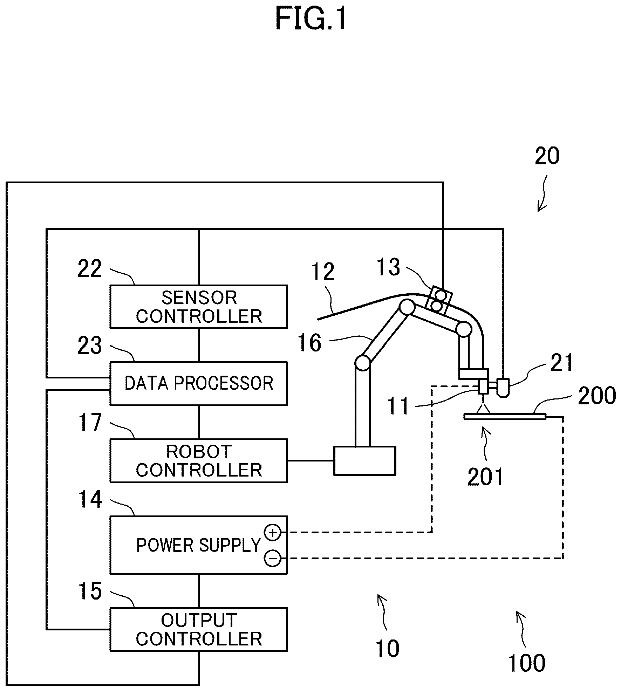

FIG. 1 is a schematic view of a configuration of a welding system of the present embodiment. A welding system 100 includes a welding apparatus 10 and an appearance inspection apparatus 20.

The welding apparatus 10 includes a welding torch 11, a wire feeder 13, a power supply 14, an output controller 15, a robot 16, and a robot controller 17. Electric power supplied from the power supply 14 to a welding wire 12 held by the welding torch 11 generates arc between the tip of the welding wire 12 and a workpiece 200, and heat is applied to the workpiece 200 to perform arc welding. Although the welding apparatus 10 includes other components and facilities such as a pipe and a gas cylinder for supplying shielding gas to the welding torch 11, such components are not illustrated and described for convenience of explanation. The power supply 14 may also be referred to as a welding power supply.

The output controller 15 is connected to the power supply 14 and the wire feeder 13 and controls a welding output of the welding torch 11, i.e., the electric power supplied to the welding wire 12 and power supply time, according to predetermined welding conditions. The output controller 15 also controls the speed and amount of the welding wire 12 fed from the wire feeder 13 to the welding torch 11. The welding conditions may be directly inputted to the output controller 15 via an input unit (not shown), or may be selected from a welding program read from a recording medium.

The robot 16, which is a known articulated robot, holds the welding torch 11 at the tip and is connected to the robot controller 17. The robot controller 17 controls the motion of the robot 16 so that the tip of the welding torch 11, in other words, the tip of the welding wire 12 held by the welding torch 11, moves to a desired position along a predetermined welding path.

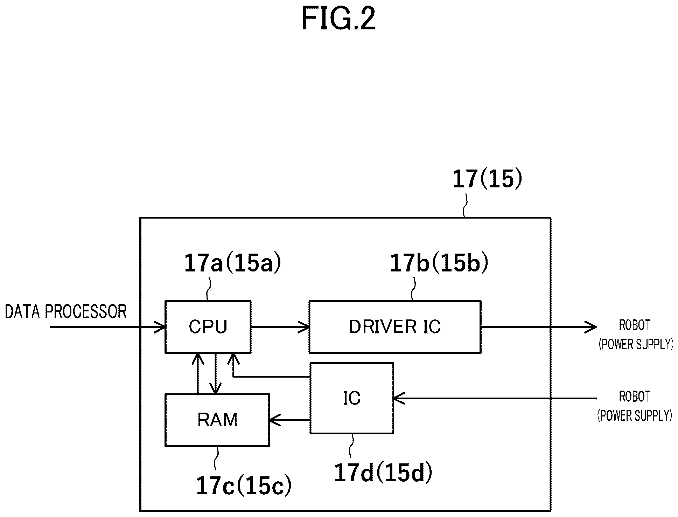

FIG. 2 is a schematic view of a hardware configuration of a robot processor. The robot controller 17 includes at least a central processing unit (CPU) 17a, a driver integrated circuit (IC) 17b, random access memory (RAM) 17c, and an integrated circuit (IC) 17d.

In normal operation, the IC 17d receives signals outputted from rotation detectors (not shown) provided on a plurality of articulated shafts of the robot 16. The outputted signals are processed by the IC 17d and inputted to the CPU 17a. The CPU 17a transmits a control signal to the driver IC 17b based on the signals inputted from the IC 17d and the rotation rate of the articulated shafts set in a predetermined program stored in the RAM 17c. The driver IC 17b controls the rotation of servo motors (not shown) connected to the articulated shafts based on the control signals from the CPU 17a.

As will be described later, when the shape of a weld 201 is determined to be bad by a first determination unit 28 of a data processor 23 of the appearance inspection apparatus 20, the CPU 17a of the robot controller 17 that received the determination result stops the motion of the robot 16 or moves the robot 16 so that the welding torch 11 moves to a predetermined initial position.

The output controller 15 also has the same configuration as the robot controller 17. That is, the output controller 15 includes at least a CPU 15a, a driver IC 15b, a RAM 15c, and an IC 15d.

In a normal operation, the IC 15d receives a signal corresponding to the output of the power supply 14. The signal is processed by the IC 15d and inputted to the CPU 15a. The CPU 15a transmits a control signal to the driver IC 15b based on the signals inputted from the IC 15d and the output of the power supply 14 set in a predetermined program stored in the RAM 15c. The driver IC 15b controls the output of the power supply 14, and by extension the welding output of the welding torch 11, based on the control signal from the CPU 15a.

As will be described later, when the shape of the weld 201 is determined to be bad by the first determination unit 28 of the appearance inspection apparatus 20, the CPU 15a of the output controller 15 that received the determination result stops the output of the power supply 14. Thus, the welding output of the welding torch 11 is stopped.

Both of the output controller 15 and the robot controller 17 may include other components than those shown in FIG. 2. For example, read only memory (ROM) or a hard disk drive (HDD) may be included as a storage device.

The appearance inspection apparatus 20 includes a shape measurement unit 21, a sensor controller 22, and a data processor 23. The shape measurement unit 21 is attached to the robot 16 or the welding torch 11 to measure the shape of the weld 201 of the workpiece 200. The configuration of the appearance inspection apparatus 20 will be described in detail later.

FIG. 1 shows an arc welding apparatus configured to perform arc welding as the welding apparatus 10, but the welding apparatus 10 is not particularly limited to the arc welding apparatus. For example, the welding apparatus 10 may be a laser welding apparatus configured to perform laser welding. In this case, a laser head (not shown) connected to a laser oscillator (not shown) via an optical fiber (not shown) is attached to and held by the robot 16 in place of the welding torch 11. In the following description, the welding torch 11 and the laser head may be collectively referred to as a welding head 11.

[Configuration of Appearance Inspection Apparatus]

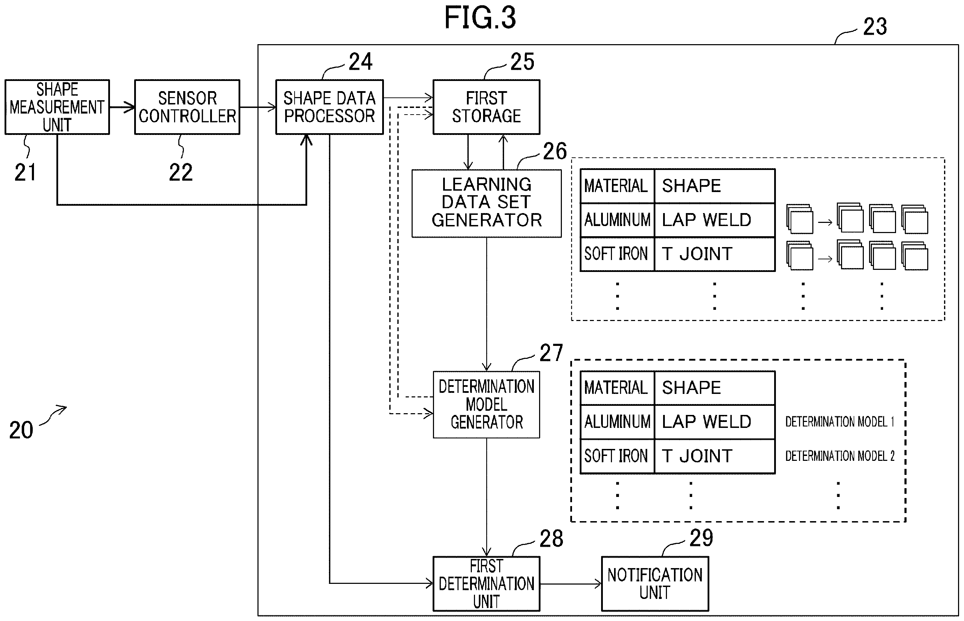

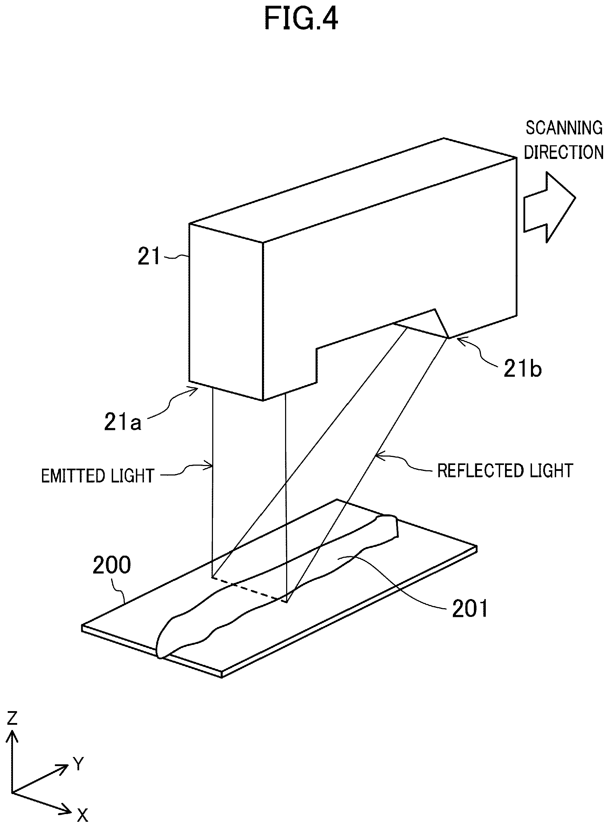

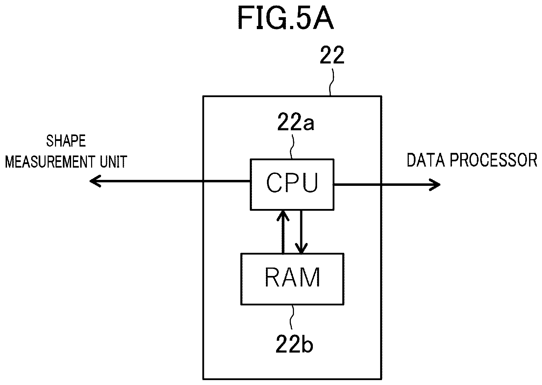

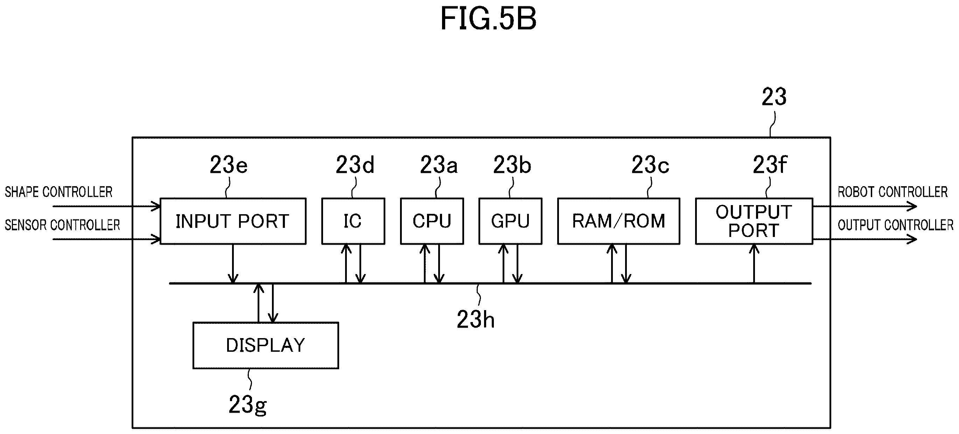

FIG. 3 is a functional block diagram of the appearance inspection apparatus, and FIG. 4 is a schematic view of measurement of the shape of a weld bead by the shape measurement unit. FIG. 5A is a schematic view of a hardware configuration of the sensor controller, and FIG. 5B is a schematic view of a hardware configuration of the data processor.

The shape measurement unit 21 is, for example, a three-dimensional shape measurement sensor including a laser beam source 21a capable of scanning the surface of the workpiece 200 (see FIG. 4) and a camera (not shown) configured to capture an image of a reflection trajectory (will be hereinafter referred to as a shape line) of a laser beam projected onto the surface of the workpiece 200 or a light receiving sensor array 21b (see FIG. 4).

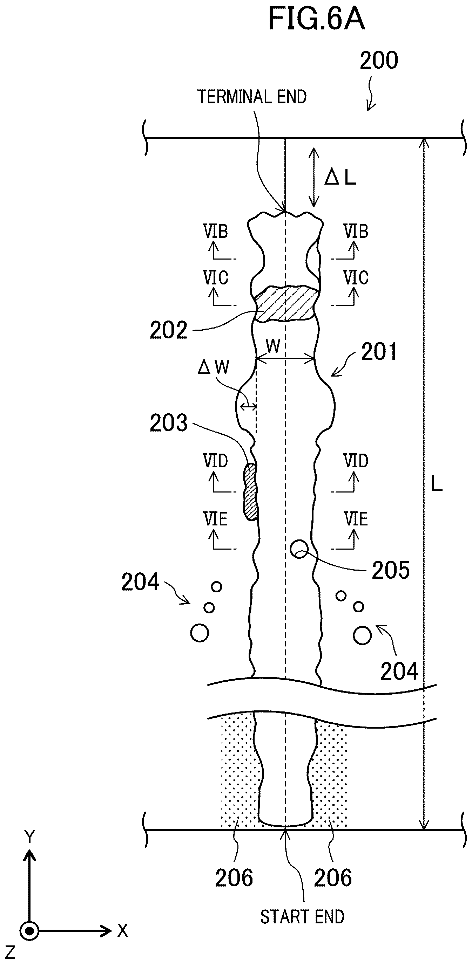

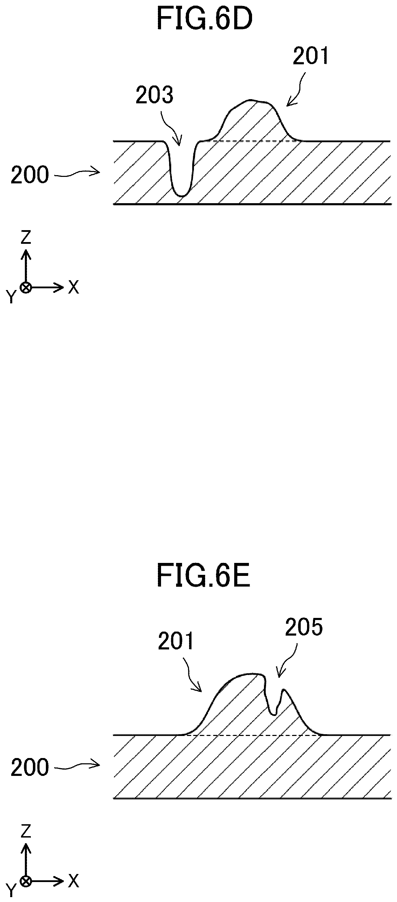

As shown in FIG. 4, the shape measurement unit 21 scans a predetermined region including the weld 201 and its periphery with the laser beam (emitted light) and captures an image of the emitted light reflected by the surface of the workpiece 200 by the light receiving sensor array 21b to measure the three-dimensional shape of the weld 201. The weld 201 is a so-called weld bead formed in a direction along a welding line set in advance by a welding program. In the following description, the direction along the welding line may be referred to as a Y direction (see FIG. 6A). A direction orthogonal to the Y direction on the surface of the workpiece 200 on which the weld 201 is formed may be referred to as an X direction. A direction of the height of the weld 201 with respect to the surface of the workpiece 200 may be referred to as a Z direction. The Z direction is orthogonal to the X direction and the Y direction.

In the present specification, objects being "orthogonal," "parallel," or "the same" are orthogonal, parallel, or the same to the degree that allows manufacturing tolerances and assembly tolerances of the components constituting the welding system 100, machining tolerances of the workpiece 200, and variations in the travel speed of the robot 16. This does not mean that the objects are orthogonal, parallel, or the same in a strict sense.

In the example shown in FIG. 4, the light emitted from the laser beam source 21a is applied to multiple points in the width direction of the weld 201, in this case, the X direction. The laser beam is reflected from the multiple points and captured by the light receiving sensor array 21b. The shape measurement unit 21 held by the robot 16 travels at a predetermined speed in the Y direction. During the travel, the light is emitted at predetermined time intervals to irradiate the weld 201 and its periphery, and the light receiving sensor array 21b captures an image of the reflection of the light each time the light is emitted.

As described above, the shape measurement unit 21 is configured to measure the shape of, not only the weld 201, but also its periphery, in a predetermined range. This is for determining whether spatters 204 and smuts 206 described later (see FIG. 6A) are present or not.

The term "measurement resolution" refers to a distance between measurement points adjacent to each other in shape data measured by the shape measurement unit 21. For example, the measurement resolution in the X direction is a distance between measurement points adjacent to each other in the X direction. The measurement resolution in the X direction is set according to the capability of the shape measurement unit 21, mainly of the light receiving sensor array 21b, and more specifically, the size in the X direction of each sensor included in the light receiving sensor array 21b and the distance between the sensors.

The measurement resolution is set in each of the X, Y, and Z directions. As will be described later, the measurement resolution in the Y direction varies depending on the travel speed of the robot 16 or the sampling frequency of the light receiving sensor array 21b.

When simply referred to as a "resolution," it means an interval between coordinate points adjacent to each other in multiple pieces of point group data of the weld 201 acquired by the shape measurement unit 21. As will be described later, the shape data is reconstructed in accordance with the shape of the weld 201. The resolution of the shape data before the reconstruction is the same as the measurement resolution described above. The resolution of the reconstructed shape data may be different from the measurement resolution. In the example shown in this specification, the X-direction resolution of the shape data is the same as the measurement resolution in the X direction. However, the Y-direction resolution of the shape data may be different from the measurement resolution in the Y direction. The resolution is set in each of the X, Y, and Z directions.

As shown in FIG. 5A, the sensor controller 22 includes at least a CPU 22a and RAM 22b. In the sensor controller 22, the CPU 22a transmits a control command to the shape measurement unit 21 to control the operation of the shape measurement unit 21. The control command transmitted from the CPU 22a to the shape measurement unit 21 includes, for example, conditions for inspection by the shape measurement unit 21 and a command to start or stop the measurement by the shape measurement unit 21. The RAM 22b stores preset inspection conditions. The RAM 22b may store other types of data. The sensor controller 22 may include other components than those shown in FIG. 5A. For example, ROM or an HDD may be included as the storage device.

The data processor 23 receives the point group data of the shape line acquired by the shape measurement unit 21 as the shape data and processes the shape data.

As shown in FIG. 3, the data processor 23 includes a plurality of functional blocks. Specifically, the data processor 23 includes a shape data processor 24, a first storage 25, a learning data set generator 26, a determination model generator 27, a first determination unit 28, and a notification unit 29.

As shown in FIG. 5B, the data processor 23 includes, as hardware, at least a CPU 23a, a graphics processing unit (GPU) 23b, RAM/ROM 23c, an IC 23d, an input port 23e, an output port 23f, and a data bus 23h. The data processor 23 includes a display 23g.

The data processor 23 shown in FIG. 5B has the same hardware configuration as a known personal computer (PC). The functional blocks in the data processor 23 shown in FIG. 3 are implemented by running predetermined software in various devices shown in FIG. 5B, particularly the CPU 23a and the GPU 23b. Although FIG. 5B shows an example in which various devices are connected to the single data bus 23h, two or more data buses may be provided depending on the purpose, as in the case of an ordinary PC.

The shape data processor 24 of the data processor 23 has the function of removing noise from the shape data acquired by the shape measurement unit 21. The reflectance of the laser beam emitted from the shape measurement unit 21 varies depending on the material of the workpiece 200. An excessive reflectance causes halation as noise, affecting the shape data. Thus, the shape data processor 24 is configured to perform a noise filtering process on the software. The noise can also be removed by an optical filter (not shown) provided for the shape measurement unit 21 itself. Combined use of the optical filter and the filtering process on the software can provide high quality shape data. This can improve the quality of a determination model of a learning data set described later, and whether the shape of the weld 201 is good or bad can be determined with high accuracy.

The noise removal function of the shape data processor 24 is mainly implemented by the IC 23d of the data processor 23. However, the present invention is not limited to this example, and the noise may be removed from the shape data by the GPU 23b of the data processor 23, for example.

The shape data processor 24 corrects an inclination and distortion of a base portion of the weld 201 with respect to a predetermined reference plane, for example, an installation surface of the workpiece 200, by statistically processing the point group data. The shape data processor 24 may also perform, for example, edge enhancement correction, by enhancing the periphery of the weld 201 to emphasize the shape and location of the weld 201.

The shape data processor 24 extracts feature values of the shape data in accordance with the shape of the workpiece 200 or inspection items for the shape of the weld 201. In this case, one or more feature values corresponding to one or more inspection items are extracted for a piece of shape data. The extracted feature values are associated with the shape data for use in subsequent data processing. The feature values are particular specifications extracted from the shape data. Typical examples thereof include a length, width, and height from the reference plane of the weld 201, and a difference in length, width, and height between a plurality of points in the weld 201. However, the feature values are not particularly limited to such specifications, and are appropriately set according to the details to be evaluated in terms of the inspection items.

The shape data processor 24 is configured to be able to convert/correct the resolution of the acquired shape data. The conversion/correction of the resolution of the shape data will be described in detail later.

The CPU 23a of the data processor 23 mainly implements the functions of edge enhancement correction, feature value extraction, and conversion/correction of the resolution of the shape data processor 24. However, the present invention is not particularly limited to this example, and the IC 23d or the GPU 23b may perform part or all of the edge enhancement correction.

The first storage 25 stores shape data of the weld 201 of a different workpiece 200 processed before the welding of the workpiece 200 as an evaluation target. The first storage 25 stores the shape data experimentally acquired in advance before welding the actual workpiece 200. In the following description, the shape data acquired in advance may be referred to as sample shape data.

The sample shape data includes non-defective data about a good shape of the weld 201 to be evaluated and defective data about a shape with some defects. The defective data is processed into multiple pieces of learning data by changing the number and locations of shape defects and labelling the shape defects with types of the shape defects. The defective data after the labelling and the non-defective data are collectively used as a learning data set before data augmentation. Needless to say, the shape data of the weld 201 of the other workpiece 200 and the shape data of the weld 201 of the target workpiece 200 are acquired from similar welds 201 of the workpieces 200 having the similar shape and being made of the same material.

For the acquisition of the sample shape data, the conditions for the inspection by the shape measurement unit 21 are fixed. However, the inspection conditions may be changed for each material or shape of the workpiece 200.

The learning data set generator 26 reads the sample shape data generated by the shape data processor 24 and stored in the first storage 25 and classifies the data by material and shape of the workpiece 200. The sample shape data may be classified by inspection item of the weld 201. In this case, the same shape data may be included in different inspection items. The learning data set generator 26 generates a learning data set for each material and shape of the workpiece 200 based on the feature value associated with the sample shape data, i.e., generates a group of learning data which is inputted to a determination model described later to improve the determination accuracy of the determination model. For example, the materials and shapes of the workpiece 200 are sorted into a matrix to determine classification categories, and the learning data sets are classified in correspondence with the categories (see FIG. 3). Examples of the shapes of the workpiece 200 include a butt weld and lap weld of plates, a T j oint, and a cross joint.

The learning data set generator 26 performs data augmentation on the sample shape data read from the first storage 25 to generate the learning data set. Specifically, the data augmentation is executed by changing one or more feature values associated with the sample shape data and/or changing the position of the shape defect in the sample shape data of the weld 201. A procedure for generating the learning data set will be described in detail later.

The function of the learning data set generator 26 is mainly implemented by the CPU 23a of the data processor 23. However, the present invention is not particularly limited to this example, and the GPU 23b may implement part of the function.

The determination model generator 27 generates a determination model based on a criterion set for each of the inspection items of the weld 201 set for each material and shape of the workpiece 200. The generated determination model is represented as, for example, a combination of two or more discriminators each of which is weighed. The determination model is, for example, a known object detection algorithm expressed by a convolutional neural network (CNN).

The determination model generator 27 inputs, among the plurality of learning data sets, the learning data set corresponding to the material and shape of the workpiece 200 to each of the determination models generated for each material and shape of the workpiece 200, and repeats the learning to improve the determination accuracy of each of the determination models. In this case, the determination models are generated according to the classification categories shown in FIG. 3. The learning is repeated until the accuracy rate, recall rate, and precision of the determination model satisfy preset values.

The determination model can be generated in a shorter time with higher accuracy when the non-defective data and the defective data in the sample shape data are suitably selected and used according to the material and shape of the workpiece 200. Likewise, the determination model can be generated in a shorter time with higher accuracy for each inspection item of the weld 201 when the non-defective data and the defective data in the sample shape data are suitably selected and used according to the inspection items.

The first determination unit 28 determines whether the shape of the weld 201 is good or bad, i.e., whether the shape satisfies a predetermined criterion, based on the shape data of the weld 201 on which the processes such as noise removal and edge enhancement have been done by the shape data processor 24 and the determination model corresponding to the selected inspection item among the determination models generated by the determination model generator 27.

Before the determination of whether the shape of the weld 201 is good or bad, the determination model is reinforced by learning using the learning data set. Specifically, as for the selected inspection item, the learning data set generated by the learning data set generator 26 is inputted to the determination model generated by the determination model generator 27, and the determination result is manually checked by an operator such as a welder. When the type of the weld defect does not match the learning data, annotation is executed. The annotation refers to a process of tagging the presence of the shape defect identified by visually checking the actual weld 201 together with the type of the shape defect to a corresponding part of the shape data. This annotation is basically manually performed.

By performing the annotation, whether the shape defect is present and the type of the shape defect are revised in the learning data. Based on the result of the annotation, the learning data set is regenerated or a new learning data set is generated, and the relearning of the determination model is performed using the annotated learning data set. By repeating these processes one or more times, the determination model is reinforced by learning.

As will be described later, correction of the resolution is performed in advance on the learning data set as necessary. Appropriate resolution correction allows the learning data set to be used for reinforcement of the determination model by learning.

However, as will be described later, the shape defect has a variety of modes. In practice, which mode the shape defect included in the shape data has is calculated in terms of probability. If the probability is equal to or higher than a predetermined value, the shape defect is determined to be present, and the type of the shape defect is identified. This will be described in detail later.

For example, the degree of coincidence between the type of the shape defect annotated in the learning data and the type of the shape defect included in the shape data of the weld 201 is determined by probability. When the probability exceeds a predetermined threshold, the type of the shape defect included in the shape data of the weld 201 is identified.

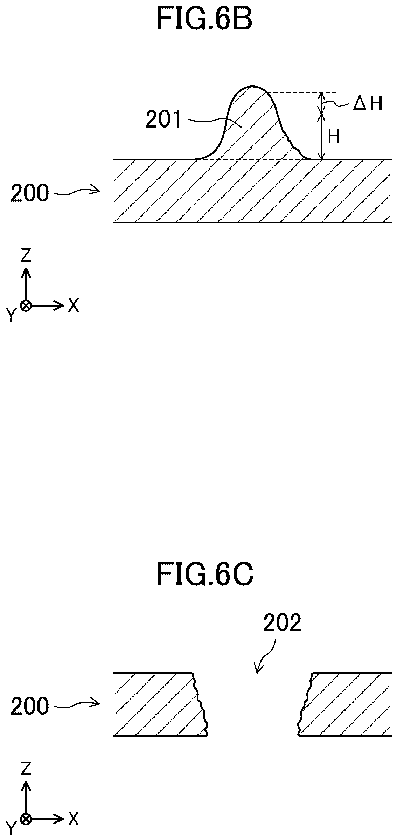

The first determination unit 28 outputs the following information. Specifically, the first determination unit 28 outputs whether the shape defect is present or not, and outputs, if the shape defect is present, the type, number, size, and location of the shape defect in the weld 201. When the number of the shape defects exceeds a threshold according to a predetermined determination criterion, the first determination unit 28 outputs the result of the determination of whether the shape of the weld 201 is good or bad. The threshold varies depending on the type and size of the shape defect. For example, if five or more spatters described later (see FIG. 6A) having a diameter of 5 $\mu$m or more are present, the shape of the weld 201 is determined to be bad. If one or more holes (see FIGS. 6A and 6C) are present, the shape of the weld 201 is determined to be bad. These are merely examples and can be changed as appropriate in accordance with the above-described determination criterion and the threshold.

The threshold for determining the shape defect and a format for displaying the shape defect can be optionally set. For example, the shape defect may be displayed in red if identified as the spatter 204, or in yellow if identified as a hole 202 (see FIG. 6A). If the presence or absence of the spatters 204 and the upper limit number of the spatters 204 are set as the inspection items, a portion recognized as the spatter 204 may be displayed in a color different from its background, and the probability that the portion is the spatter 204 may be classified by color. Thus, the welder or a system administrator can easily recognize the presence or absence of the shape defects and the degree of distribution of the shape defects at a glance. For example, the probability of the degree of coincidence may be colored in green if the probability is 30% or less, or in red if the probability is 70% or more. Needless to say, this classification of the probability ranges by color and the definition of the colors can be arbitrarily set.

The shape of the weld 201 is inspected for a variety of inspection items, and whether the shape is good or bad is determined for each inspection item. The product is finally determined to be good only when the shape of the weld 201 has satisfied all the inspection items for which the determination is necessary.

The notification unit 29 is configured to notify the output controller 15, the robot controller 17, the welder, or the system administrator of the result of the determination by the first determination unit 28. For example, the display 23g of the data processor 23 corresponds to the notification unit 29. For the notification, the determination result may be shown on the display 23g or a display unit (not shown) of the welding system 100 and/or may be outputted from a printer (not shown). If only a simple notification of the final determination result is sufficient, voice notifying the result may be outputted from an audio output unit which is not shown. In a preferred embodiment, the notification unit 29 notifies not only the final determination result, but also the determination result for each inspection item. The notification in this manner allows the welder or the system administrator to specifically realize what kind of defect the weld 201 has.

If the result of the determination by the first determination unit 28 is positive, i.e., the shape of the weld 201 is determined to be good, the welding system 100 continuously welds a portion 201 to be welded next of the same workpiece 200, or a similar portion 201 to be welded of a next workpiece 200.

If the result of the determination by the first determination unit 28 is negative, i.e., the shape of the weld 201 is determined to be bad, the output controller 15 stops the welding output of the welding torch 11, and the robot controller 17 stops the motion of the robot 16 or operates the robot arm 16 so that the welding torch 11 moves to a predetermined initial position.

[Procedure for Generating Learning Data Set]

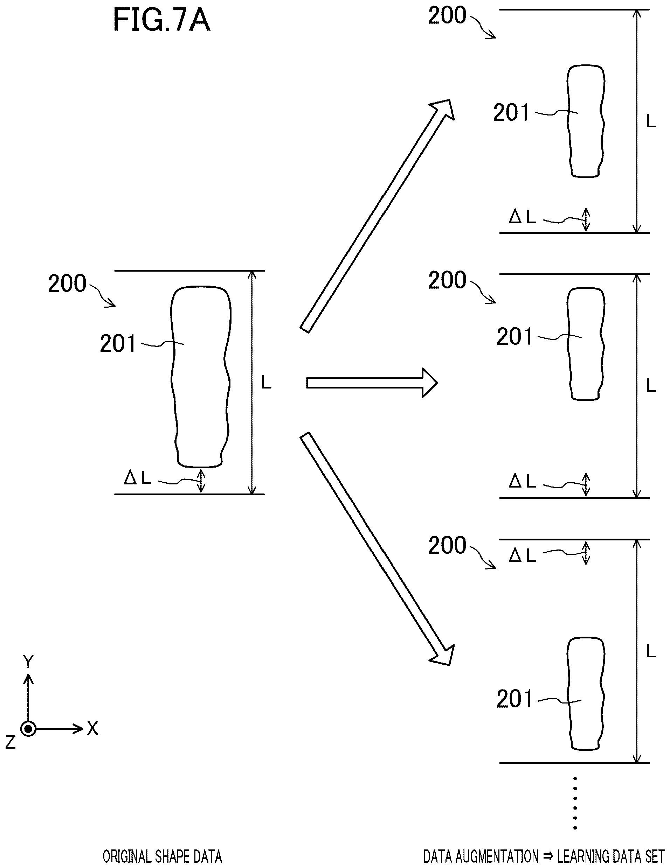

FIGS. 6A to 6E show examples of the shape defect generated in the weld, and FIGS. 7A to 7C show examples of the procedure for generating the learning data set. FIGS. 6A to 6E show the shape of the weld 201 which is butt-welded. FIG. 6A shows a planar shape, and FIGS. 6B to 6E show cross-sectional views taken along line VIB-VIB or line VIE-VIE of FIG. 6A.

As shown in FIGS. 6A to 6E, when the workpiece 200 is arc-welded or laser-welded, the weld 201 may have various kinds of shape defect depending on, for example, poor setting of the welding conditions and low quality of the workpiece 200 used. For example, the weld 201 may partially melt off (a through hole formed in the workpiece 200 when the weld 201 partially melts off the workpiece 200 may be hereinafter referred to as a hole 202), or an undercut 203 may be formed. The undercut 203 means a defective portion that is formed at an edge of a weld bead and is dented from the surface of the workpiece 200. The length, width, and height from the reference plane of the weld 201 may vary from their design values L, W, and H beyond allowable ranges ∆L, ∆W, and ∆H. Further, when droplets (not shown) generated at the tip of the welding wire 12 move to the workpiece 200, some of the droplets or fine particles of molten metal of the workpiece 200 may be scattered to generate the spatters 204. When the workpiece 200 is a galvanized steel sheet, the sheet may partially evaporate from the weld 201 to leave a pit 205. When the workpiece 200 or the welding wire 12 is made of an aluminum-based material, smut 206 may be generated near the weld 201.

The pit 205 opens at the surface of the weld bead, and the smut 206 is a black soot-like product that adheres to the vicinity of the weld bead. The pit 205 and the smut 206, and the above-described hole 202, undercut 203, and spatter 204 as well, are examples of the modes (types) of the shape defect.

As described above, the shape defect of the weld 201 has various modes, for each of which the criterion is required to perform the inspection in accordance with the criterion. For the hole 202 or the undercut 203, whether the shape is good or bad needs to be determined not only by its presence or absence, but also by setting, for example, a contrast ratio to or a height difference from the periphery of the weld 201, to identify the hole 202 or the undercut 203. For the spatters 204, for example, it is necessary to obtain its average diameter and determine whether the shape is good or bad by the number of the spatters 204 having an average diameter equal to or greater than a predetermined value per unit area. The number of inspection items and the criterion for determining whether the shape of the weld 201 is good or bad are changed or increased depending on the material and portion to be welded of the workpiece 200 and specifications required by the customer.

The criterion for determining whether the shape defect is present from the shape data varies depending on the material and shape of the workpiece 200. As described above, the reflectance of the laser beam varies depending on the material of the workpiece 200, and for example, the luminance level and contrast of the shape data also vary. For welding straight portions having the same length, the shape of the bead of the weld 201 may vary due to the influence of gravity depending on the shape of the workpiece 200.

Thus, the determination model generator 27 needs to generate the determination models using a large amount of learning data for each material and shape of the workpiece 200. That is, a large amount of shape data of the weld 201 suitable as the learning data needs to be acquired for each material and shape of the workpiece 200. However, acquiring the sample shape data necessary for each material and shape of the workpiece 200 in advance involves enormous number of man-hours, which is inefficient.

According to the present embodiment, the learning data set generator 26 classifies the sample shape data read from the first storage 25 by material and shape of the workpiece 200, and performs data augmentation on each of the classified pieces of sample shape data to generate a plurality of learning data sets, i.e., a group of learning data required for the generation of the determination model.

For example, as shown in FIG. 7A, the length and position of the weld 201, which are feature values, in the original sample shape data are varied to generate multiple pieces of data as the learning data sets. In the example shown in FIG. 7A, multiple pieces of shape data are generated in each of which the length of the weld 201 is smaller than the reference value L beyond the allowable range ∆L. However, the shape data is not particularly limited to this example, and shape data in which the length is greater than the reference value L beyond the allowable range ∆L is also generated.

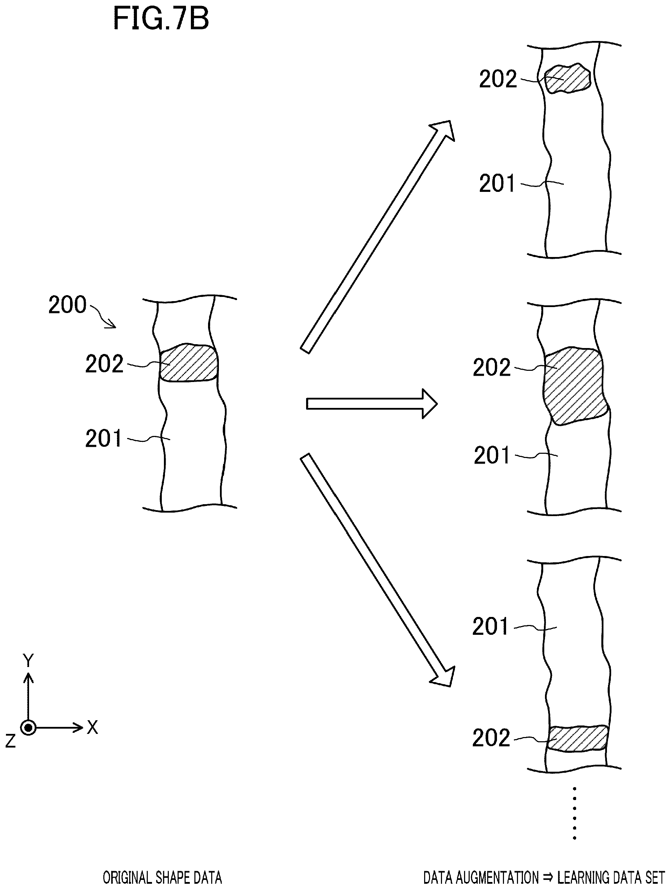

In another example, as shown in FIG. 7B, the size and position of the hole 202 in the original sample shape data are varied to generate multiple pieces of data as the learning data sets. In this case, the height from the reference plane and the difference in height between two or more points in the weld 201 are extracted as the feature values, and are varied.

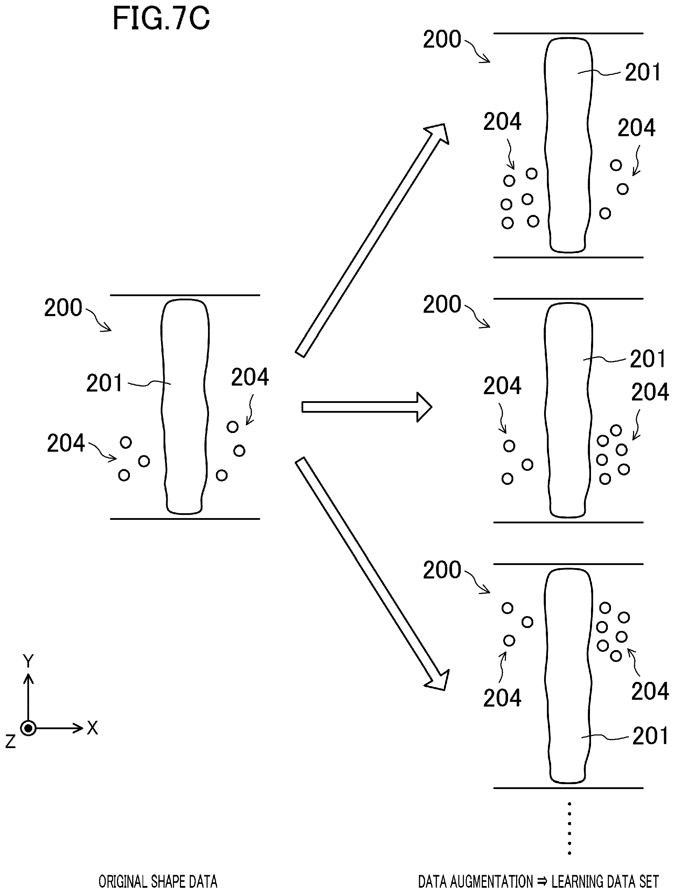

In still another example, as shown in FIG. 7C, the number and position of the spatters 204 in the original sample shape data are varied to generate multiple pieces of data as the learning data sets.

When similar feature values are extracted around the weld 201 and the learning data set is generated based on the feature values, whether the spatters 204 and the smut 206 are present beyond the predetermined allowable range can be determined.

[Procedure for Weld Appearance Inspection]

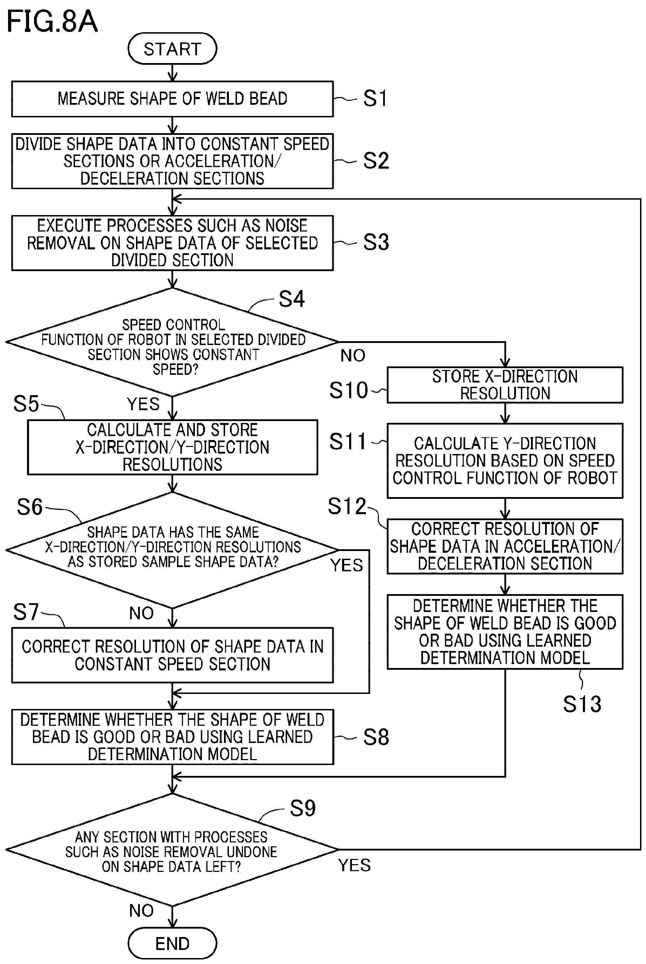

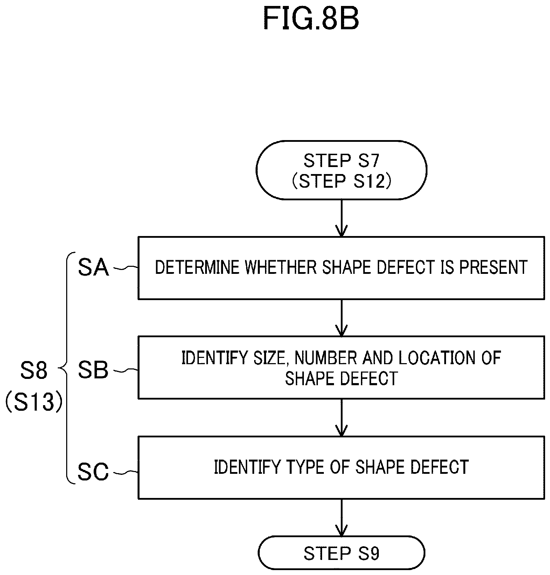

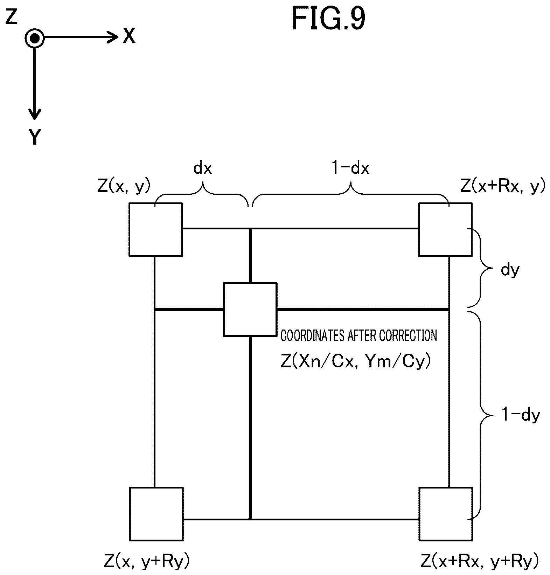

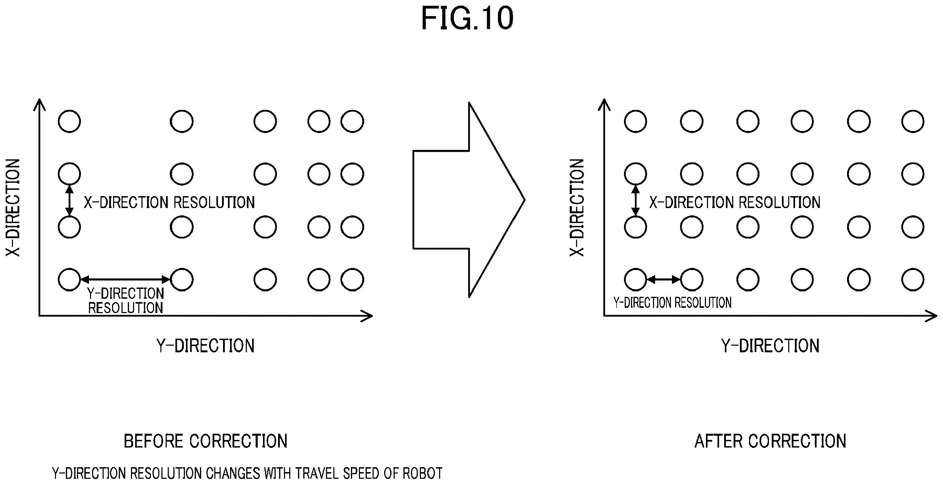

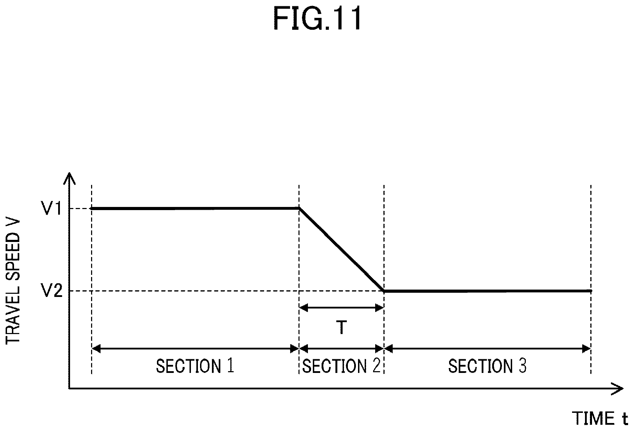

FIG. 8A is a flowchart of a procedure for weld appearance inspection, and FIG. 8B is a flowchart of a procedure for determining whether the shape of a weld bead is good or bad. FIG. 9 is a conceptual diagram illustrating a procedure for deriving coordinate points of the shape data in conversion/correction of the resolution. FIG. 10 is a conceptual diagram illustrating how the positions of the coordinate points in the shape data change before and after the conversion/correction of the resolution in the acceleration/deceleration section. FIG. 11 is a schematic view of an example of the speed control function of the robot in the acceleration/deceleration section.

For the appearance inspection of the weld 201 using the learning data set prepared in advance, the conditions for the inspection by the shape measurement unit 21 need to be the same as the inspection conditions for acquiring the shape data used for generating the learning data, that is, the sample shape data.

As described above, the inspection conditions are often changed depending on the production takt time and the inspection accuracy of the workpiece 200. In this case, however, the shape data of the weld 201 and the feature values extracted from the shape data vary depending on the inspection conditions, and the first determination unit 28 may fail to correctly determine whether the shape of the weld 201 is good or bad.

Thus, in the present embodiment, the resolution of the shape data is corrected instead of setting the same inspection conditions for acquiring the shape data as the conditions for acquiring the sample shape data in advance. Specifically, focusing on the measurement resolution of the shape measurement unit 21, the shape data is corrected so that the resolution of the shape data to be measured has the same value as the resolution of the sample shape data acquired in advance. This correction is the conversion/correction of the resolution described above. This allows the first determination unit 28 to correctly determine whether the shape of the weld 201 is good or bad, although the inspection conditions are changed. This will be described in further detail below.

First, the shape of the weld 201 is measured by the shape measurement unit 21 (Step S1 in FIG. 8A) to acquire the shape data.

Next, the data processor 23 acquires the travel speed of the robot 16, that is, the speed of the shape measurement unit 21 scanning the weld 201 in the Y direction, from the robot controller 17. The data processor 23 divides a section scanned by the shape measurement unit 21 in the Y direction into a constant speed section and an acceleration/deceleration section based on the travel speed of the robot 16 (Step S2 in FIG. 8A). The "constant speed section" refers to a section in which the shape measurement unit 21 attached to the robot 16 travels at a constant speed in the Y direction. The "acceleration/deceleration section" refers to a section in which the shape measurement unit 21 attached to the robot 16 travels at an accelerating speed, a decelerating speed, or both accelerating and decelerating speeds, in the Y direction.

The shape data processor 24 performs the above-described processes such as edge enhancement correction and noise removal on the shape data in the section selected from the sections divided in Step S2. This section will be hereinafter referred to as a selected section (Step S3 in FIG. 8A).

Next, the data processor 23 determines whether the selected section for which Step S3 has been executed is the constant speed section. This determination is made based on whether the speed control function of the robot 16 in the selected section indicates a constant speed, that is, a speed that is constant with respect to time (Step S4 in FIG. 8A). The speed control function of the robot 16 is transmitted from the robot controller 17 to the data processor 23 in response to a request from the data processor 23.

<When Selected Section is Constant Speed Section>

If the determination result in Step S4 is positive, that is, the selected section is the constant speed section, the shape data processor 24 calculates the resolution in the X direction (hereinafter referred to as an X-direction resolution) and resolution in the Y direction (hereinafter referred to as a Y-direction resolution) of the shape data acquired in Step S1, and stores the calculated resolutions in the first storage 25 (Step S5 in FIG. 8A).

As described above, the X-direction resolution corresponds to a distance between the measurement points adjacent to each other in the X direction. In general, the scanning width of the laser beam emitted by the shape measurement unit 21 is constant. The Y-direction resolution in the constant speed section is expressed by the following Formula (1).

$$Ry = 1000\,V\,/\,60\,F = 50\,V\,/\,3\,F \quad (1)$$

where Ry (mm) is the Y-direction resolution of the shape data in the selected section, V (m/min) is the travel speed of the robot 16, and F (Hz) is the measurement frequency of the shape measurement unit 21.

That is, in the Y direction, the shape is measured at every period of 1/F. In the X direction, multiple measurement points are measured at once at every period of 1/F over the scanning width of the laser beam. The X-direction resolution of the shape data is usually determined according to the size and interval of pixels of a camera which is not shown or the light receiving sensor array 21b in the shape measurement unit 21. This is also the case when the selected section is the constant speed section or the acceleration/deceleration section.

The X-direction resolution and measurement frequency F of the shape measurement unit 21 are transmitted from the sensor controller 22 to the data processor 23 in response to a request from the data processor 23.

After Step S5 is executed, the shape data processor 24 determines whether the X-direction resolution and Y-direction resolution of the shape data in the selected section have the same values as the X-direction resolution and Y-direction resolution of the sample shape data previously acquired and stored in the first storage 25 (Step S6 in FIG. 8A).

If the determination result of Step S6 is positive, the process proceeds to Step S8, and the first determination unit 28 determines whether the shape of the weld 201 in the selected section is good or bad. The details of Step S8 will be described later.

If the determination result in Step S6 is negative, the shape data processor 24 converts/corrects the resolution of the shape data in the selected section (Step S7 in FIG. 8A).

As shown in FIG. 9, the resolution is converted/corrected by using, relative to the height in the Z direction of a predetermined coordinate point $(x, y)$, the height in the Z direction of each of other coordinate points $(x + Rx, y)$, $(x, y + Ry)$, and $(x + Rx, y + Ry)$ adjacent to the predetermined coordinate point $(x, y)$. Rx (mm) is the X-direction resolution of the shape data in the selected section.

In the following description, the height in the Z direction at a coordinate point $(x, y)$, i.e., Z coordinates, is represented by $Z(x, y)$. The origin of the coordinate point $(x, y)$ is set at, for example, the start end of the weld 201. In this case, the origin of the Z coordinates $Z(x, y)$ is set with reference to the surface of the workpiece 200 near the start end.

The resolution is converted/corrected by the following procedure. First, as shown in Formulae (2) and (3), an X-direction resolution coefficient Cx and a Y-direction resolution coefficient Cy are calculated.

$$Cx = Rx / Rx_0 \quad (2)$$

$$Cy = Ry / Ry_0 \quad (3)$$

where $Rx_0$ is the X-direction resolution of the sample shape data, and $Ry_0$ is the Y-direction resolution of the sample shape data. The X-direction resolution $Rx_0$ and the Y-direction resolution $Ry_0$ are stored in the first storage 25 in advance.

Next, for each of the XY coordinates reconstructed with the resolution at the acquisition of the sample shape data, $Z(Xn/Cx, Ym/Cy)$ is calculated as the Z coordinates to satisfy Formula (4).

$$Z(Xn/Cx, Ym/Cy) = (1 - dx) \times (1 - dy) \times Z(x, y)$$

$$+ dx \times (1 - dy) \times Z(x + Rx, y)$$

$$+ (1 - dx) \times dy \times Z(x, y + Ry)$$

$$+ dx \times dy \times Z(x + Rx, y + Ry) \quad (4)$$

where n is a variable corresponding to each point of the point group data in the X direction, is an integer, and satisfies $1 \leq n \leq N$ (N is the number of point groups in the X direction), m is a variable corresponding to each point of the point group data in the Y direction, is an integer, and satisfies $1 \leq m \leq M$ (M is the number of point groups in the Y direction),

dx is a value obtained by dividing the distance in the X direction between the coordinate point $(x, y)$ and the coordinate point $(Xn/Cx, Ym/Cy)$ by the distance in the X direction between the coordinate point $(x, y)$ and the coordinate point $(x + Rx, y)$, and dy is a value obtained by dividing the distance in the Y direction between the coordinate point $(x, y)$ and the coordinate point $(Xn/Cx, Ym/Cy)$ by the distance in the Y direction between the coordinate point $(x, y)$ and the coordinate point $(x, y + Ry)$.

That is, the ratio of dx to (1 - dx) shown in FIG. 9 is the ratio of the distance in the X direction between the coordinate point $(x, y)$ and the coordinate point $(Xn/Cx, Ym/Cy)$ to the distance in the X direction between the coordinate point $(x + Rx, y)$ and the coordinate point $(Xn/Cx, Ym/Cy)$. Likewise, the ratio of dy to (1 - dy) is the ratio of the distance in the Y direction between the coordinate point (x, y) and the coordinate point (Xn/Cx, Ym/Cy) to the distance in the Y direction between the coordinate point (x, y + Ry) and the coordinate point (Xn/Cx, Ym/Cy).

The height in the Z direction at the coordinate point (Xn/Cx, Ym/Cy) is derived based on the heights in the Z direction at four points around the coordinate point (Xn/Cx, Ym/Cy), that is, the coordinate points (x, y), (x + Rx, y), (x, y + Ry), and (x + Rx, y + Ry) before reconstruction.

Z (Xn/Cx, Ym/Cy) as the coordinates after the correction shown in Formula (4) is calculated for all the point groups in the selected section, and thus, the conversion/correction of the resolution of the shape data is completed.

After Step S7 is executed, the process proceeds to Step S8, and the first determination unit 28 determines whether the shape of the weld 201 in the selected section is good or bad using the shape data having the resolution converted/corrected.

After Step S8 is executed, the data processor 23 determines whether any section where the process of Step S3 is unexecuted is present among the divided sections of the shape data (Step S9 in FIG. 8A).

If the determination result of Step S9 is negative, the process returns to Step S3. Then, the section where the process of Step S3 is unexecuted is selected, the process of Step S3 is executed, and the series of steps are repeated until the determination result of Step S9 turns to be positive.

If the determination result in Step S9 is positive, the shape data of the measured weld bead has no section where the preprocessing such as the noise removal is unexecuted, and no divided section where the shape evaluation is unexecuted is left. Thus, the appearance inspection of the weld 201 ends.

<When Selected Section is Acceleration/Deceleration Section>

If the determination result in Step S4 is negative, that is, the selected section is the acceleration/deceleration section, the data processor 23 acquires the X-direction resolution of the shape data acquired in Step S1 from the sensor controller 22 and stores the acquired resolution in the first storage 25 (Step S10 in FIG. 8A).

Next, the shape data processor 24 calculates the Y-direction resolution of the shape data acquired in Step S1 based on the speed control function of the robot 16 (Step S11 in FIG. 8A). The speed control function of the robot 16 is transmitted from the robot controller 17 to the shape data processor 24 of the data processor 23 in response to a request from the data processor 23. The speed control function of the robot 16 may be temporarily transmitted to and stored in the first storage 25, and then transmitted to the shape data processor 24.

Further, the shape data processor 24 performs the conversion/correction of the resolution of the shape data in the selected section.

The resolution of the shape data at the time of acceleration or deceleration, particularly the Y-direction resolution, will be described with reference to FIG. 10 (Step S11 in FIG. 8A).

In general, for the appearance inspection of one weld 201, the scanning frequency and scanning width of the laser beam are rarely changed. As described above, when the direction along the welding line is the Y direction, the X direction is a direction intersecting with the direction along the welding line. The laser beam of the shape measurement unit 21 for measuring the shape travels in the Y direction at the travel speed of the tip of the robot 16 (hereinafter, simply referred to as the travel speed of the robot 16) to periodically scan the weld in the X direction across the welding line. Thus, the X-direction resolution Rx of the shape data can be considered to be constant in many cases in each of the constant speed section and the acceleration/deceleration section.

The Y-direction resolution Ry changes in accordance with the travel speed V of the robot 16. When the selected section is the constant speed section, the measurement frequency F and the movement speed V are constants, and the Y-direction resolution Ry is also a constant as is clear from Formula (1).

When the selected section is the acceleration/deceleration section, for example, when the robot 16 is traveling at an accelerating speed, the interval in the Y direction between the measurement points adjacent to each other increases with time. When the robot 16 is traveling at a decelerating speed, the interval in the Y direction between the measurement points adjacent to each other decreases with time. As a result, for example, as shown in the left graph in FIG. 10, the Y-direction resolution changes between the adjacent measurement points in the Y direction. When the shape of the weld 201 is evaluated based on such point group data (shape data), an accurate result cannot be obtained as described above.

Thus, when the selected section is the acceleration/deceleration section, the Y-direction resolution needs to be corrected to a form corresponding to the speed control function of the robot 16. Specifically, the Y-direction resolution Ry(t) (mm) is expressed as shown in Formula (5).

$$Ry(t) = 1000\ V(t)\ /\ 60\ F = (50/3F) \times V(t) \quad (5)$$

where V(t) (m/min) is the speed control function of the robot 16, and F (Hz) is the measurement frequency of the shape measurement unit 21. As will be described later, V(t) is described by a k-th order function (k is an integer of one or more) of time t (sec).

A resolution coefficient $Cy_m(t)$ at the m-th coordinate point in the Y direction from the origin is expressed by Formula (6).

[Math 1]

$$Cy_m(t) = \int_0^{Tm} \left( \frac{Ry(t)}{n \times Ry0} \right) dt \qquad \cdots (6)$$

where Tm is time taken to travel from the origin to the m-th coordinate point.

Next, the Z coordinates as the coordinates after the correction are calculated to satisfy Formula (7) for each point of the XY coordinates reconstructed with the resolution at the acquisition of the sample shape data.

$$Z(Xn/Cx, Ym/Cy_m(t)) = (1 - dx) \times (1 - dy) \times Z(x, y)$$

$$+ dx \times (1 - dy) \times Z(x + Rx, y)$$

$$+ (1 - dx) \times dy \times Z(x, y + Ry)$$

$$+ dx \times dy \times Z(x + Rx, y + Ry) \quad (7)$$

Formula (7) is the same as Formula (4) except that the reconstructed Y coordinates $Ym/Cy_m(t)$ are described as the function of time t.

$Z(Xn/Cx, Ym/Cy_m(t))$ shown in Formula (7) are calculated for all the point groups included in the selected section, and the conversion/correction of the resolution of the shape data is completed (Step S12 in FIG. 8A).

After Step S12 is executed, the process proceeds to Step S13, and the first determination unit 28 determines whether the shape of the weld 201 in the selected section is good or bad using the shape data after the conversion/correction of the resolution. The details of Step S13 will be described later.

After Step S13 is executed, the data processor 23 determines whether any section where the process of Step S3 is unexecuted is present among the divided sections of the shape data (Step S9 in FIG. 8A).

If the determination result of Step S9 is negative, the process returns to Step S3. Then, the section where the process of Step S3 is unexecuted is selected, the process of Step S3 is executed, and the series of steps are repeated until the determination result of Step S9 turns to be positive.

If the determination result in Step S9 is positive, no section in which the shape is not evaluated is left. Thus, the appearance inspection of the weld 201 ends.

As shown in FIG. 11, an example in which the shape data is divided into three sections (sections 1 to 3) will be described.

As is apparent from FIG. 11, the section 1 and the section 3 are the constant speed sections. Thus, the appearance of the weld 201 is inspected by executing Steps S1 to S8 of FIG. 8A.

The section 2 is the deceleration section. Specifically, the travel speed V (m/min) of the robot 16 monotonously decreases from V1 to V2 (< V1) in period T (sec). Thus, the speed control function V(t) of the robot 16 in the section 2 is expressed in the form shown in Formula (8).

$$V(t) = A \times t + B = ((V2 - V1)/T) \times t + V1 \quad (8)$$

Specifically, the speed control function V(t) is a linear function of time t, a linear coefficient A of time t is (V2 - V1)/T, and a constant B is V1.

In this case, the data processor 23 acquires various types of information characterizing the speed control function V(t) from the robot controller 17. For example, when the speed control function V(t) is a k-th order function (k is an integer of one or more) of time t, each coefficient value of t to $t^k$ and the value of the constant B are acquired. In the section 2, the appearance inspection of the weld 201 is performed by executing Steps S1 to S4 and S10 to S13 in FIG. 8A.

<Procedure for Determining Whether Shape of Weld Bead is Good or Bad>

A procedure for determining whether the shape of the weld bead (the weld 201) is good or bad shown in FIG. 8B includes the same processes as Steps S8 and S13 of FIG. 8A, and will be described together.

Each of Steps S8 and S13 in FIG. 8A is divided into sub steps SA to SC shown in FIG. 8B. First, the first determination unit 28 determines whether the shape data in the selected section includes a shape defect (Step SA). A determination model used in this step is previously reinforced by learning using a learning data set as described above. For the reinforcement by learning, the learning data set may be subjected to the resolution correction in advance.

The first determination unit 28 identifies the size and number of the shape defects and the location of each shape defect in the weld 201 (Step SB). Further, the first determination unit 28 identifies the type of the shape defect (Step SC).

In Step SC, as described above, the type of the shape defect is identified in consideration of the shape and size of the shape defect and the location of the shape defect in the weld 201. In this case, for example, the probability that the shape defect is the spatter 204 is calculated, and the shape defect is identified as the spatter 204 if the probability is equal to or higher than a predetermined value (e.g., 70%).

The final result of the determination of the shape of the weld 201 is transmitted to the notification unit 29 or the display 23g. If the shape is determined to be bad, the shape data obtained by the shape measurement unit 21, i.e., the shape of the weld 201, is displayed on the display 23g as point group data.

When all the welds 201 included in one workpiece 200 are determined to be good, the workpiece 200 is determined to be a non-defective product and is sent to the subsequent process or is shipped as a non-defective product.

Several measures can be taken when a defect is found in one or more welds 201 included in one workpiece 200. For example, after the appearance inspection of all the welds 201 included in the workpiece 200, the inspection result is stored, and the workpiece 200 is discarded as a defective product. In this case, the inspection result is stored in, for example, the first storage 25 of the data processor 23. However, the present invention is not limited to this example. When the defect is found in the weld 201, the workpiece 200 may be discarded as a defective product.

For example, after the appearance inspection of all the welds 201 included in the workpiece 200, the inspection result may be stored, and the workpiece 200 may proceed to a repair process. In the repair process, the weld 201 determined to be defective is rewelded.

For example, after the appearance inspection of all the welds 201 included in the workpiece 200, the inspection result may be stored, and the welder may visually check the defective welds 201 again. Whether the weld 201 is repairable is determined by the visual check. If the workpiece 200 is determined to be repairable, the workpiece 200 proceeds to the repair process, and the defective weld 201 is rewelded.

[Advantages]

As described above, the appearance inspection apparatus 20 of the present embodiment inspects the appearance of the weld 201 of the workpiece 200.

The appearance inspection apparatus 20 includes at least the shape measurement unit 21 that is attached to the robot 16 and configured to measure the three-dimensional shape of the weld 201 along a welding line and the data processor 23 configured to process the shape data acquired by the shape measurement unit 21.

The data processor 23 includes at least the shape data processor 24 configured to perform at least correction of the resolution of the shape data. The data processor 23 further includes the learning data set generator 26 configured to generate a plurality of learning data sets by performing data augmentation on multiple pieces of sample shape data acquired in advance by the shape measurement unit 21, and the determination model generator 27 configured to generate a determination model for determining whether the shape of the weld 201 is good or bad using the plurality of learning data sets.

The data processor 23 further includes the first determination unit 28 configured to determine whether the shape of the weld 201 is good or bad based on the shape data corrected by the shape data processor 24 and one or more determination models generated by the determination model generator 27.

The appearance inspection apparatus 20 configured as described above can accurately evaluate the three-dimensional shape of the weld 201 and can correctly determine whether the shape of the weld 201 is good or bad, although the inspection conditions are changed according to the production takt time and the required inspection accuracy.

A single workpiece 200 usually includes a large number of welds 201. In this case, the workpiece 200 often includes various types of welds 201 having different shapes, and the inspection conditions are changed as appropriate in accordance with the shapes of the welds 201.

According to the present embodiment, although one workpiece 200 includes the welds 201 having different inspection conditions, the three-dimensional shape of each weld 201 can be accurately evaluated, and whether the shape of the weld 201 is good or bad can be correctly determined.

The shape data processor 24 corrects the resolution of the shape data acquired by the shape measurement unit 21 based on the conditions for the inspection by the shape measurement unit 21. The inspection conditions are, for example, the measurement resolution, measurement frequency, and scanning speed of the shape measurement unit 21. As described above, the scanning speed of the shape measurement unit 21 corresponds to the scanning speed of the laser beam in the X direction, the travel speed V of the robot 16, or the speed control function V(t) of the robot 16.

Thus, the resolution of the shape data can be converted/corrected easily and accurately.

The sample shape data is acquired at the measurement resolution, measurement frequency, and scanning speed of the shape measurement unit 21 that are determined in advance. The shape data processor 24 corrects the resolution of the shape data acquired by the shape measurement unit 21 to the same value as the resolution of the sample shape data.

As described above, the determination model is reinforced by learning based on each of the plurality of learning data sets. The learning data sets are generated based on the sample shape data which is shape data experimentally acquired in advance before welding the actual workpiece 200. The resolution of the shape data is corrected to the same value as the resolution of the sample shape data. In this manner, the shape defects included in the sample shape data, and by extension in each of the learning data set and the shape data, such as the hole 202 and the spatter 204, can have shape features matched. Thus, whether the shape of the weld 201 is good or bad can be determined reliably and accurately using the learned determination model.

The appearance inspection apparatus 20 further includes the sensor controller 22 configured to store conditions for the inspection by the shape measurement unit 21 and transmit the stored inspection conditions to the data processor 23. When the direction along the welding line is the Y direction, the sensor controller 22 transmits the measurement resolution in the X direction intersecting with the Y direction and the Z direction which is the height direction of the weld 201 and the measurement frequency to the data processor 23.

The data processor 23 receives the travel speed V of the robot 16 or the speed control function V(t) of the robot 16 from the robot controller 17 configured to control the motion of the robot 16.

Thus, the resolution of the shape data can be converted/corrected easily and accurately.

The shape data processor 24 corrects the value of the shape data in the Z direction based on the X-direction resolution and Y-direction resolution of the shape data.

When the shape measurement unit 21 measures the three-dimensional shape of the weld 201 while the robot 16 is traveling along the welding line at a constant speed, the Y-direction resolution is determined based on the measurement frequency of the shape measurement unit 21 and the travel speed V of the robot 16.

When the shape measurement unit 21 measures the three-dimensional shape of the weld 201 while the robot 16 is traveling at an accelerating speed, a decelerating speed, or both the accelerating and decelerating speeds in a predetermined section along the welding line, the Y-direction resolution is determined based on the measurement frequency F of the shape measurement unit 21 and the speed control function V(t) of the robot 16. The speed control function V(t) is described by a k-th order function of time t. However, the present invention is not limited to this example, and the speed control function V(t) may be, for example, a sine wave function or a cosine wave function. That is, the speed control function V(t) is a function depending on time t.

Thus, the resolution of the shape data can be easily and accurately converted/corrected although the scanning speed of the shape measurement unit 21 is changed in various ways.

The learning data set generator 26 classifies the multiple pieces of sample shape data acquired in advance by the shape measurement unit 21 by material and shape of the workpiece 200, and performs data augmentation on the classified pieces of sample shape data to generate a plurality of learning data sets.

The determination model generator 27 generates a determination model for each material and shape of the workpiece 200 using the plurality of learning data sets.

With the appearance inspection apparatus 20 configured in this manner, a required number of learning data sets can be generated although the amount of the sample shape data is small, and the determination model can be provided with enhanced accuracy. This allows accurate determination of whether the shape of the weld 201 is good or bad. Further, a large amount of sample shape data is no longer necessary, and the number of man-hours required for determining whether the shape is good or bad can be significantly reduced. The shape defect of the weld 201 can be automatically detected without manually setting a complicated criterion for the determination. The multiple pieces of sample shape data are classified by material and shape of the workpiece 200 prior to the generation of the learning data sets, allowing efficient generation of the learning data sets.

The data processor 23 further includes the first storage 25 configured to store at least the sample shape data used for generating the plurality of learning data sets. In this case, the learning data set generator 26 reads the sample shape data stored in the first storage 25 to generate the plurality of learning data sets.

Thus, the generation of the learning data sets and the subsequent generation of the determination model can be smoothly performed.

The data processor 23 further includes the notification unit 29 configured to notify the result of the determination by the first determination unit 28.

This allows the welder or the system administrator to know in real time during the welding of the workpiece 200 whether a failure has occurred at the weld 201 or not. If necessary, measures to continue the welding of the workpiece 200 or not can be taken. This can reduce the cost of the welding process.

The learning data set generator 26 generates the learning data sets based on one or more feature values extracted from the sample shape data. The feature value is extracted by the shape data processor 24.

The learning data sets are generated using the feature value extracted from the sample shape data. This can simplify the generation of the learning data sets without deteriorating the accuracy of the determination model.

The learning data set generator 26 performs the data augmentation by changing one or more feature values extracted from the sample shape data and/or changing the position of the shape defect in the sample shape data.

The learning data sets are generated based on the one or more feature values extracted from the sample shape data. Thus, the learning data sets can be generated with improved efficiency, and the number of man-hours can further be reduced. The learning data sets can be efficiently generated by a simple process of changing the feature values and/or the position of the shape defect.

The learning data set generator 26 may classify the multiple pieces of sample shape data by inspection item for the weld 201, and may perform the data augmentation on the classified pieces of sample shape data to generate the plurality of learning data sets.

When determining whether the shape of the weld 201 is good or bad, the first determination unit 28 determines whether the inputted shape data includes the shape defect. In this determination, the learning data set is generated using the sample shape data including non-defective data having no shape defect and defective data having some shape defect. In the learning data set, the defective data is processed such that the type of the shape defect is identified and the shape defect is labelled with the identified type. The determination model is previously reinforced by learning using the learning data set.

When the shape data includes the shape defect, the first determination unit 28 identifies the number and size of the shape defects and the location of each shape defect in the weld 201 and a predetermined region around the weld 201.

The first determination unit 28 identifies the type of each shape defect. In this identification, the number and size of the shape defects and the location of each shape defect in the weld 201 are referred to. The type of the shape defect is calculated in terms of probability, and the type of the shape defect is determined when the probability is equal to or higher than a predetermined threshold. The type of the shape defect is not limited to those shown in FIGS. 6A to 6E. When the dimension of the weld 201 does not satisfy a predetermined criterion for non-defective products, it is also regarded as the shape defect. The criterion for the dimension of the non-defective product can be set in any of the X direction, Y the direction, and the Z direction.

As described above, the first determination unit 28 determines or identifies each of the plurality of items about the shape of the weld 201, and finally determines whether the shape of the weld 201 is good or bad based on the results. This allows accurate and reliable evaluation of whether the shape of the weld 201 is good or bad.

The determination model generator 27 may generate the determination model for determining whether the shape of the weld 201 is good or bad for each inspection item of the weld 201 using the plurality of learning data sets.

The learning data set generator 26 may classify each of the multiple pieces of sample shape data into a piece of sample shape data of a particular portion of the weld 201 in which the determination of the shape defect is more difficult than in the other portion and a piece of sample shape data of the other portion, and may separately perform the data augmentation on the pieces of sample shape data to generate the plurality of learning data sets.

Alternatively, when generating the determination model for each material and shape of the workpiece 200 using the plurality of learning data sets, the determination model generator 27 may separately generate the determination model corresponding to the particular portion of the weld 201 and the determination model corresponding to the other portion.

This allows the determination of whether the shape defect is present and the identification of the type of the shape defect with accuracy equal to or more than a predetermined level, although in the particular portion of the weld 201 where the determination and/or the identification is more difficult than in the other portion. This allows accurate determination of whether the shape of the weld 201 is good or bad in the appearance inspection.

In the present embodiment, the determination model used to determine whether the shape defect is present is the same as the determination model for identifying the type of the shape defect. However, the determination models may be provided separately.

The welding system 100 of the present embodiment includes the welding apparatus 10 configured to weld the workpiece 200 and the appearance inspection apparatus 20.

The welding system 100 configured in this manner can inspect the shape of the weld 201 with high accuracy and a small number of man-hours. This can reduce the cost of the welding process.

The welding apparatus 10 includes at least the welding head 11 (welding torch 11) for applying heat to the workpiece 200, the robot 16 for holding and moving the welding head 11 (welding torch 11) to a desired position, the output controller 15 for controlling the welding output of the welding head 11 (welding torch 11), and the robot controller 17 for controlling the motion of the robot 16.

When the first determination unit 28 of the appearance inspection apparatus 20 determines that the shape of the weld 201 is bad, the output controller 15 stops the welding output of the welding head 11 (welding torch 11), and the robot controller 17 stops the motion of the robot 16 or operates the robot 16 so that the welding head 11 (welding torch 11) moves to a predetermined initial position.

The welding system 100 configured in this manner can stop the next welding if the shape of the weld 201 is bad, and can reduce the frequent production of defective products. Based on the result of the determination by the first determination unit 28 acquired for each inspection item, a failed part of the welding system 100 can be presumed, and a cause of the failure can be quickly removed, shortening downtime of the welding system 100.

A method for correcting the shape data according to the present embodiment includes measuring the three-dimensional shape of the weld 201 by the shape measurement unit 21 moving together with the robot 16 to acquire sample shape data for generating a plurality of learning data sets. The method further includes measuring the three-dimensional shape of the weld 201 by the shape measurement unit 21 moving together with the robot 16 to acquire the shape data.

The method for correcting the shape data further includes correcting, when the resolution of the shape data acquired by the shape measurement unit 21 is different from the resolution of the sample shape data, the shape data by the shape data processor 24 so that the resolution of the shape data acquired by the shape measurement unit 21 has the same value as the resolution of the sample shape data.

In this manner, the shape defects included in the sample shape data, and by extension in each of the learning data set and the shape data, such as the hole 202 and the spatter 204, can have shape features matched. Thus, whether the shape of the weld 201 is good or bad can be determined reliably and accurately when the shape data after the conversion/correction of the resolution is inputted to the learned determination model.

A method for appearance inspection of the weld 201 according to the present embodiment includes at least measuring the three-dimensional shape of the weld 201 by the shape measurement unit 21 moving together with the robot 16 to acquire sample shape data for generating a plurality of learning data sets.

The method further includes generating one or more determination models for determining whether the shape of the weld 201 is good or bad by the determination model generator 27 using the plurality of learning data sets.

The method further includes measuring the three-dimensional shape of the weld 201 by the shape measurement unit 21 moving together with the robot 16 to acquire shape data.

The method for appearance inspection of the weld 201 further includes correcting, when the resolution of the shape data acquired by the shape measurement unit 21 is different from the resolution of the sample shape data, the shape data by the shape data processor 24 so that the resolution of the shape data acquired by the shape measurement unit 21 has the same value as the resolution of the sample shape data.

The method further includes determining whether the shape of the weld 201 is good or bad by the first determination unit 28 based on the shape data having the resolution corrected by the shape data processor 24 and the one or more determination models generated by the determination model generator 27.

According to the present embodiment, the three-dimensional shape of the weld 201 can be evaluated with high accuracy, and whether the shape of the weld 201 is good or bad can be correctly determined, although the inspection conditions are changed according to the production takt time and the required inspection accuracy.

Further, according to the present embodiment, when the appearance of a plurality of welds 201 included in one workpiece 200 is inspected, the shape of each weld 201 can be evaluated with high accuracy although the inspection conditions are changed each time in accordance with the shape of the weld 201. For example, although the weld 201 has a curved portion or a recessed portion that may limit the movement range of the robot 16, the shape of the weld 201 can be accurately evaluated without being affected by the change in the inspection conditions.

The welder can set the inspection conditions separately for a portion to be inspected at a high speed in consideration of the production takt time and a portion to be inspected at a low speed in consideration of the inspection accuracy. For example, the inspection conditions can be set separately for one weld 201 or for a plurality of welds 201 included in one workpiece 200. In either case, the present embodiment allows accurate evaluation of the shape of the weld 201 and correct determination of whether the shape of the weld 201 is good or bad.

Each of the steps (Steps S8 and S13 in FIG. 8A) of determining whether the shape of the weld 201 is good or bad by the first determination unit 28 further includes the following sub steps.

Specifically, the sub steps include determining whether the weld 201 has the shape defect based on the shape data inputted from the shape data processor 24 and the determination model reinforced by learning (sub step SA in FIG. 8B), identifying the number and size of shape defects and the location of each shape defect with respect to the weld 201 (sub step SB in FIG. 8B), and identifying the type of each shape defect (sub step SC in FIG. 8B).

The first determination unit 28 determines whether the shape of the weld 201 is good or bad based on the results of the determination and the identification in each of the sub steps SA to SC.

This allows accurate and reliable evaluation of whether the shape of the weld 201 is good or bad.

In the appearance inspection of the weld 201, whether or not to provide the acceleration/deceleration section or the travel speed of the robot 16 is determined according to the shape of the weld 201 and the required production takt time. Thus, for the appearance inspection of the weld 201, the resolution of the shape data needs to be converted/corrected by the shape data processor 24 according to the shape of the weld 201 and the required production takt time. This will be described below by way of Examples.

[Example 1]

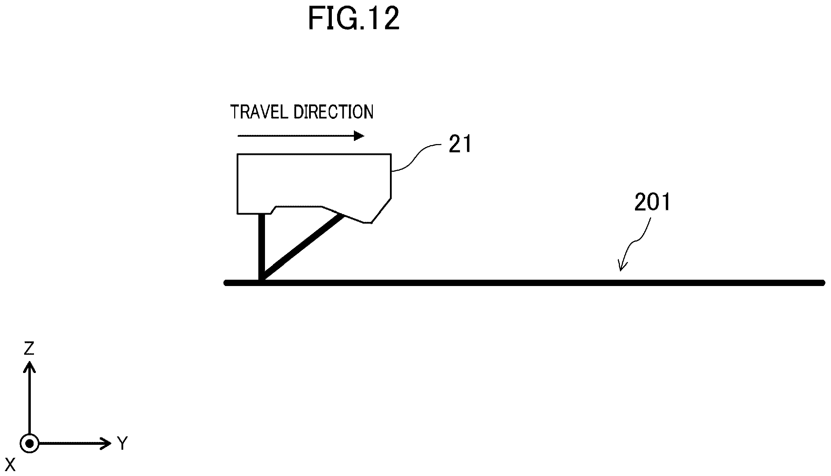

FIG. 12 is a schematic view of the appearance inspection of a weld according to Example 1. For convenience of explanation, the shape of the shape measurement unit 21 is schematically shown. Other components than the shape measurement unit 21 and the weld 201 are not shown. In FIG. 12 and the subsequent drawings, the same components as those described in the first embodiment are denoted by the same reference numerals, and are not described in detail.

In the example shown in FIG. 12, the appearance of the weld 201, which was a linear weld bead, was inspected while moving the shape measurement unit 21 at a constant speed. The measurement range (scanning range) of the shape measurement unit 21 was 5 mm in the X direction and 45 mm in the Y direction.

The measurement frequency F was 1000 Hz, the travel speed V of the robot 16 (corresponding to the above-described constant speed) was 9 m/min, and the measurement resolution of the shape measurement unit 21 in the X direction was 0.05 mm (= 50 $\mu$m). In this case, the X-direction resolution Rx of the shape data was also 0.05 mm (= 50 $\mu$m). The values of the measurement frequency F and the travel speed V were substituted into Formula (1), and the Y-direction resolution Ry of the shape data was calculated to be 0.15 mm (= 150 $\mu$m).

The sample shape data had the X-direction resolution $Rx_0$ of 0.025 mm (= 25 $\mu$m) and the Y-direction resolution $Ry_0$ of 0.1 mm (= 100 $\mu$m). Thus, the resolutions were converted/corrected in the following procedure. The values were substituted into Formulae (2) and (3) to calculate the X-direction resolution coefficient Cx to be 2 and the Y-direction resolution coefficient Cy to be 1.5.

Suppose the point group data measured by the shape measurement unit 21 has the number of rows (the number of point group rows) in the X direction Nx, the number of rows in the Y direction Ny, the length in the X direction Lx, and the length in the Y direction Ly, the numbers of point group rows Nx and Ny are expressed by Formulae (9) and (10).

$$Nx = (Lx/Rx) + 1 \quad (9)$$

$$Ny = (Ly/Ry) + 1 \quad (10)$$

The lengths Lx and Ly were 5 mm and 45 mm, respectively, and the numbers of point group rows Nx and Ny were 101 and 301, respectively. In Formulae (9) and (10), the second term on the right side was obtained by adding a row including the origin.

The numbers of point group rows Nx' and Ny' after the reconstruction of the shape data were obtained by replacing the X-direction resolution Rx of the shape data in Formula (9) with the X-direction resolution $Rx_0$ of the sample shape data and replacing the Y-direction resolution Ry of the shape data in Formula (10) with the Y-direction resolution $Ry_0$ of the sample shape data. Specifically, the numbers of point group rows Nx' and Ny' were 201 and 451, respectively. That is, the original shape data was reconstructed into point group data constituted of 201 point group rows in the X direction and 451 point group rows in the Y direction.

With respect to each point of the reconstructed point group data, Z (Xn/Cx, Ym/Cy) as the coordinates after the correction shown in Formula (4) was calculated based on Formula (4), the values of the Z coordinates were corrected, and the conversion/correction of the resolutions was completed. Further, the first determination unit 28 determined whether the shape of the weld 201 was good or bad using the learned determination model.

Correcting the resolution of the shape data to the same value as the resolution of the sample shape data in this manner can reduce erroneous recognition of the shape defect by the determination model when the shape data is inputted to the determination model. Thus, although the shape data is acquired under the conditions different from the inspection conditions for acquiring the sample shape data, the shape of the weld 201 can be accurately evaluated, and whether the shape is good or bad can be correctly determined.

[Example 2]

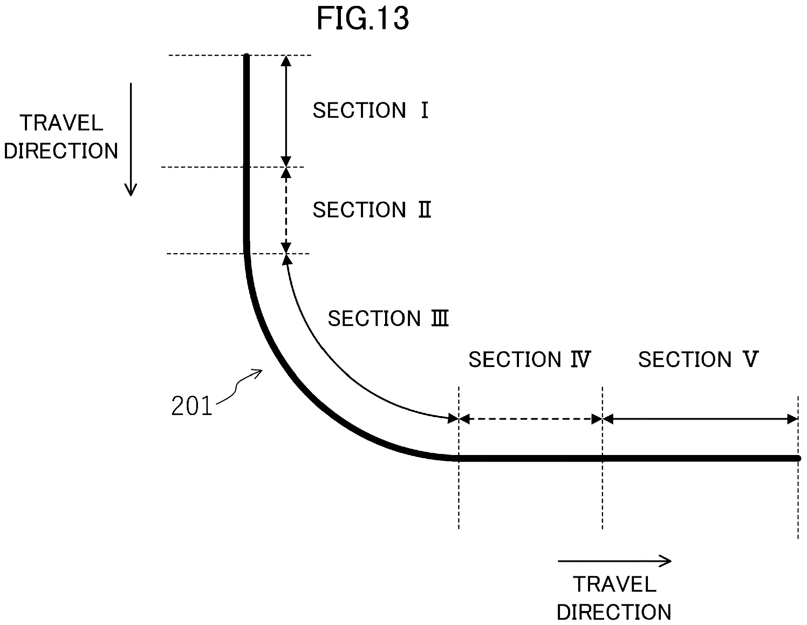

FIG. 13 is a schematic view of the planar shape of the weld according to Examples 2 and 3. The weld 201 has a substantially L-shape having a curved portion in plan view. The weld 201 is divided into sections I and II which are linear portions, a section III which is a curved portion having a substantially quadrant arc shape, and sections IV and V which are linear portions.

In this example, an appearance inspection performed on a range from the sections I to III will be described.

In the linear section I, small shape defects are less likely to occur. Thus, the appearance inspection in the section I gives priority to the reduction of the production takt time by shortening time taken to inspect the appearance over the acquisition of the shape data of the weld 201 with a higher measurement resolution.

In the curved section III, small shape defects tend to occur more frequently than in the section I. Thus, the shape data of the weld 201 needs to be acquired with a higher measurement resolution for more accurate appearance inspection in the section III.

In this example, the sections I and III were set as the constant speed sections, the travel speed V1 of the robot 16 in the section I was set to 9 m/min, and the travel speed V3 of the robot 16 in the section III was set to 3 m/min. The section II was a deceleration section in which the travel speed V decreased linearly from V1 to V3 with time t. The speed control function V(t) of the robot 16 in the section II was similar to the form shown in Formula (8).

The measurement frequency F and the X-direction resolution Rx in each of the sections I and III as the constant speed sections were set to the same value as that in Example 1, namely, 0.05 mm (= 50 $\mu$m). Further, the Y-direction resolution Ry in the section I was also set to 0.15 mm (= 150 $\mu$m), which was the same value as that in Example 1. The Y-direction resolution Ry in the section III decreased to 0.05 mm (= 50 $\mu$m) as the travel speed V decreased from V1 = 9 m/min (section I) to V3 = 3 m/min (section III).

In each of the section I and the section III as the constant speed sections, the numbers of point group rows Nx and Ny at the time of acquisition of the shape data were set to the same values as those in Example 1. That is, the numbers of point group rows Nx and Ny at the time of acquiring the shape data were 101 and 301, respectively.

In the section II as the deceleration section, the X-direction resolution Rx, the number of point group rows Nx at the time of acquiring the shape data, and the measurement frequency F were set to the same values as those in the section I and the section III. Specifically, the X-direction resolution Rx was 0.05 mm (= 50 $\mu$m), the number of point group rows Nx was 101, and the measurement frequency F was 1000 Hz.

Period T as a deceleration period in the section II (see FIG. 11) was 0.2 sec. Thus, when V1 = 9 m/min, V2 = (V3 =) 3 m/min, and T = 0.2 sec were substituted into the speed control function V(t) shown in Formula (8), the linear coefficient A of time t in Formula (8) was (V2 - V1)/T = - 30. The constant B (V1) was 9.

In this case, when Formula (8) and the measurement frequency F (Hz) were substituted into Formula (5), the Y-direction resolution Ry(t) in the section II was expressed in the form shown in Formula (11).

$$Ry(t) = (50/3F) \times V(t) = (1/60) \times (-30t + 9)$$

$$= -0.5\,t + 0.15 \quad (11)$$

The resolution coefficient $Cy_m(t)$ was obtained by substituting the Y-direction resolution Ry(t) shown in Formula (11) into Formula (6). Travel time Tm (sec) as time taken to travel the section II (target section) in this case was expressed in the form shown in Formula (12).

$$0 \leq Tm = m/F = m/1000 \leq T = 0.2 \quad (12)$$

where m represents the m-th position from the origin of the target section.

In this case, the number of point group rows Nx' after the reconstruction of the shape data was 201. This was obtained by replacing the X-direction resolution Rx of the shape data in Formula (9) with the X-direction resolution $Rx_0$ of the sample shape data. The length Lx of the X-direction resolution $Rx_0$ (0.025 mm (= 25 $\mu$m), the measurement range (scanning range) of the shape measurement unit 21 in the X direction) was 5 mm. The number of point group rows Ny' after the reconstruction of the shape data was expressed in the form shown in Formula (13).

[Math 2]

$$Ny' = \int_0^T (V(t) \cdot 50/3) \cdot dt \big/ Ryo \; + 1$$
$$= \int_0^{0.2} (V(t) \cdot 50/3) \cdot dt \big/ 0.1 \; + 1$$
$$= 201 \quad \cdots (13)$$

Thus, the original shape data was reconstructed into point group data constituted of 201 point group rows in the X direction and 201 point group rows in the Y direction, and Z (Xn/Cx, Ym/Cy$_m$(t)) as the coordinates after the correction was calculated to satisfy Formula (7) with respect to the XY coordinates (Xn, Ym).

In the present embodiment, n was an integer of one or more that satisfies $1 \leq n \leq 201$, and m was an integer of one or more that satisfies $1 \leq m \leq 201$.

Z (Xn/Cx, Ym/Cym(t)) as the coordinates after the correction shown in Formula (7) was calculated for all the point groups included in the section II. Further, (Xn/Cx, Ym/Cy$_m$(t), Z (Xn/Cx, Ym/Cy$_m$(t))) was calculated for all the coordinate points included in the section II, and the shape data of the section II with the corrected resolution was obtained by the conversion/correction of the resolution.

In each of the sections I to III, the shape data having the resolution corrected by the conversion/correction was obtained, and then the first determination unit 28 determined whether the shape of the weld 201 was good or bad using the learned determination model.

Thus, correcting the resolution of the shape data measured by the shape measurement unit 21 by the conversion/correction to the same value as the resolution of the sample shape data in this manner can reduce erroneous recognition of the shape defect by the determination model when the shape data is inputted to the determination model. The determination model is generated in advance based on each of a plurality of learning data sets generated based on the sample shape data, and is further reinforced by learning. Thus, although the shape data is acquired under the conditions different from the inspection conditions for acquiring the sample shape data, the shape of the weld 201 can be accurately evaluated, and whether the shape is good or bad can be correctly determined.

Further, also in the deceleration section (section II) in which the resolution changes with time, correcting the resolution of the shape data by the conversion/correction described above can reduce erroneous recognition of the shape defect by the determination model when the shape data is inputted to the determination model. This allows accurate evaluation of the shape of the weld 201 and correct determination of whether the shape is good or bad also in the deceleration section.

[Example 3]

In this example, appearance inspection performed on the range from the sections I to V in FIG. 13 will be described.

In the sections I to III, the resolution of the shape data was converted/corrected by the method described in Example 2, and the appearance of the weld 201 was inspected. The section V was a constant speed section in which the travel speed V (V5) was 12 m/min. Specifically, in the section V, the resolution of the shape data was converted/corrected by the same method as in the section I or the section III of Example 1 or 2, and the appearance of the weld 201 is inspected. Thus, the conversion/correction of the resolution in the section IV will be mainly described.

The section IV was an acceleration section in which the travel speed V increased linearly from V3 to V5 with time t. The speed control function V(t) of the robot 16 in the section IV as the acceleration section was the same as the form shown in Formula (8).

In the section IV, the X-direction resolution Rx of the shape data to be measured, the number of point group rows Nx at the time of acquisition of the shape data, and the measurement frequency F were set to the same values as those in the sections I and III. Specifically, the X-direction resolution Rx was 0.05 mm (= 50 $\mu$m), the number of point group rows Nx was 101, and the measurement frequency F was 1000 Hz.

The acceleration period T in the section IV was 0.2 sec. Thus, when V1 = (V3 =) 3 m/min, V2 = (V5 =) 12 m/min, and T = 0.2 sec were substituted into the speed control function V(t) shown in Formula (8), the linear coefficient A of time t in Formula (8) was (V2 - V1)/T = 45. The constant B (V3) was 3.

In this case, when Formula (8) and the measurement frequency F were substituted into Formula (5), the Y-direction resolution Ry(t) in the section IV was expressed by Formula (14).

$$\mathrm{Ry(t)} = (50/3F) \times \mathrm{V(t)} = (1/60) \times (45t + 3)$$

$$= 0.75t + 0.05 \quad (14)$$

The resolution coefficient Cy$_m$(t) was obtained by substituting the Y-direction resolution Ry(t) shown in Formula (14) into Formula (6). The travel time Tm in this case was also expressed by the form shown in Formula (12).

In this case, the number of point group rows Nx' after the reconstruction of the shape data was 201. The number of point group rows Ny' after the reconstruction of the shape data was expressed in the form shown in Formula (15).

[Math 3]

$$Ny' = \int_0^T (V(t) \cdot 50/3) \cdot dt \Big/ Ryo + 1$$
$$= \int_0^{0.2} (V(t) \cdot 50/3) \cdot dt \Big/ 0.1 + 1$$
$$= 251 \quad \cdots (15)$$

As described above, the original shape data was reconstructed into point group data constituted of 201 point group rows in the X direction and 251 point group rows in the Y direction, and Z ($Xn/Cx$, $Ym/Cy_m(t)$) was calculated to satisfy Formula (7) with respect to the XY coordinates ($Xn$, $Ym$).

In this example, n was an integer of one or more that satisfies $1 \le n \le 201$, and m was an integer of one or more that satisfies $1 \le m \le 251$.

Z ($Xn/Cx$, $Ym/Cy_m(t)$) as the coordinates after the correction shown in Formula (7) was calculated for all the point groups included in the section IV Further, ($Xn/Cx$, $Ym/Cy_m(t)$, Z ($Xn/Cx$, $Ym/Cy_m(t)$)) was calculated for all the coordinate points included in the section IV, and the shape data with corrected resolution was obtained in the section IV

After the shape data with the corrected resolution was obtained in each of the sections I to V, the first determination unit 28 determined whether the shape of the weld 201 was good or bad using the learned determination model.

In this way, when the resolution of the shape data is converted to correct the resolution of the shape data to the same value as the resolution of the sample shape data, erroneous recognition of the shape defect by the determination model can be reduced when the shape data is inputted to the determination model. Thus, although the shape data is acquired under the conditions different from the inspection conditions for acquiring the sample shape data, the shape of the weld 201 can be accurately evaluated, and whether the shape is good or bad can be correctly determined.

Further, also in the deceleration section (section II) and the acceleration section (section IV) in which the resolution changes with time, the above-described conversion/correction of the resolution of the shape data can reduce erroneous recognition of the shape defect by the determination model when the shape data is inputted to the determination model. Thus, in the deceleration section and the acceleration section, the shape of the weld 201 can be accurately evaluated, and whether the shape is good or bad can be correctly determined.

[Example 4]

It has already been described that the resolution of the shape data acquired by the shape measurement unit 21 is preferably set to the same value as the resolution of the sample shape data when the resolution is not converted/corrected. This allows accurate evaluation of the shape of the weld 201 and correct determination of whether the shape is good or bad.

When acquiring the shape data, it is preferable to keep the scanning speed of the shape measurement unit 21 in the Y direction, that is, the travel speed V of the robot 16, constant in the entire inspection section. This can keep the resolution of the shape data constant over the entire inspection section.

For this purpose, the shape data is usually acquired by the following method. First, the shape measurement unit 21 is moved at an accelerating speed from a stationary state to the start point of the inspection section. In this case, the travel speed V is set to reach a desired value when the shape measurement unit 21 reaches the start point of the measurement section. Thereafter, the shape of the weld 201 is measured while the shape measurement unit 21 is moved at a constant speed V over the entire inspection section. Thus, the resolution of the shape data is kept constant over the entire inspection section, allowing accurate evaluation of the shape of the weld 201 and correct determination of whether the shape is good or bad.

However, this method is disadvantageous in that the robot 16 needs to travel in a section other than the inspection section, that is, an approach section, and the production takt time required for the appearance inspection increases.

According to the method disclosed in the present specification, the resolution of the shape data can be converted and corrected to the same value as the resolution of the sample shape data also in the acceleration/deceleration section in which the travel speed V changes. Thus, the shape of the weld 201 can be evaluated without providing the approach section. This will be described below by way of the example.

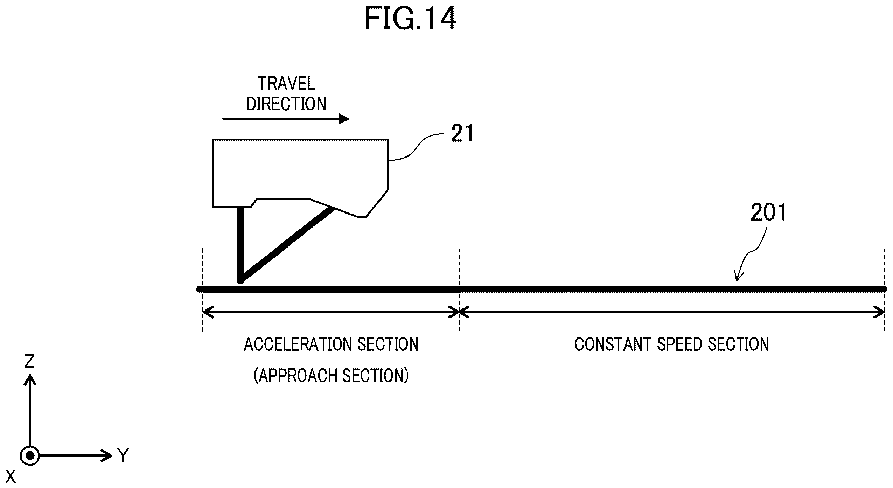

FIG. 14 schematically shows how the appearance of the weld was inspected in Example 4. The appearance of the weld 201 which was a linear weld bead was inspected by moving the shape measurement unit 21 as described below. In this example, the shape measurement unit 21 was accelerated while moving the robot 16 from a state in which the shape measurement unit 21 was stationary. The speed control function V(t) of the robot 16 in this case was the same as the form shown in Formula (8). Further, from the time point when the speed reached the speed Vc (= 9 m/min), the shape measurement unit 21 was moved at the same speed. The shape measurement unit 21 continuously measured the shape of the weld 201 for the appearance inspection from the time point when the shape measurement unit 21 started to move.

The section from the time point when the shape measurement unit 21 started to move to the time point when the speed reached the speed Vc is the acceleration section and also corresponds to the above-described approach section. Thus, without the approach section provided separately from the inspection section in the conventional method, the resolution of the shape data is converted and corrected to the same value as the resolution of the sample shape data also in the acceleration/deceleration section in which the travel speed V changes. Thus, the resolution of the acquired shape data can be corrected to the same value as the resolution of the sample shape data over the entire inspection section including the acceleration section.

In the acceleration section shown in FIG. 13, the X-direction resolution Rx of the shape data to be measured, the number of point group rows Nx at the acquisition of the shape data, and the measurement frequency F were set to the same values as those in the constant speed section shown in FIG. 13. Specifically, the X-direction resolution Rx was 0.05 mm (= 50 $\mu$m), the number of point group rows Nx was 101, and the measurement frequency F was 1000 Hz. The number of point group rows Ny of the shape data in the Y direction in the constant speed section was set to 301.

The acceleration period T in the acceleration section was 0.3 sec. Thus, when V1 = 0 m/min, V2 = (Vc =) 9 m/min, and T = 0.3 sec were substituted into the speed control function V(t) shown in Formula (8), the linear coefficient A of time t in Formula (8) was (V2 - V1)/T = 30. The constant B was 0, and the speed control function V(t) was 30t.

In this case, when Formula (8) and the measurement frequency F (Hz) were substituted into Formula (5), the Y-direction resolution Ry(t) in the acceleration section was expressed in the form shown in Formula (14).

$$Ry(t) = (50/3F) \times V(t) = (1/60) \times (30t)$$

$$= 0.5\,t \quad (16)$$

The resolution coefficient $Cy_m(t)$ was obtained by substituting the Y-direction resolution Ry(t) shown in Formula (16) into Formula (6). The travel time Tm (sec) in this case was also expressed by the form shown in Formula (12).

In this case, the number of point group rows Nx' after the reconstruction of the shape data was 201, as in the section II as the deceleration period in Example 2 and the section IV as the acceleration section in Example 3. The number of point group rows Ny' after the reconstruction of the shape data was expressed in the form shown in Formula (17).

[Math 4]

$$Ny' = \int_0^T (V(t) \cdot 50/3) \cdot dt \Big/ Ryo + 1$$
$$= \int_0^{0.3} (V(t) \cdot 50/3) \cdot dt \Big/ 0.1 + 1$$
$$= 226 \quad \cdots (17)$$

As described above, the original shape data was reconstructed into point group data constituted of 201 point group rows in the X direction and 226 point group rows in the Y direction, and Z (Xn/Cx, Ym/$Cy_m$(t)) as coordinates after the correction was calculated to satisfy Formula (7) with respect to the XY coordinates (Xn, Ym).

In this example, n was an integer of one or more that satisfies $1 \leq n \leq 201$, and m was an integer of one or more that satisfies $1 \leq m \leq 226$.

Z (Xn/Cx, Ym/$Cy_m$(t)) as the coordinates after the correction shown in Formula (7) was calculated for all the point groups included in the acceleration section. Further, (Xn/Cx, Ym/$Cy_m$(t), Z (Xn/Cx, Ym/$Cy_m$(t))) was calculated for all the coordinate points included in the acceleration section, and the resolution of the shape data was converted in the acceleration section to obtain the shape data with the corrected resolution.

After the shape data with the corrected resolution was obtained in the entire inspection section, that is, in each of the acceleration section and the constant speed section, the first determination unit 28 determined whether the shape of the weld 201 was good or bad by the learned determination model.

In this way, when the resolution of the shape data acquired by the shape measurement unit 21 is converted and corrected to the same value as the resolution of the sample shape data, erroneous recognition of the shape defect by the determination model can be reduced when the shape data is inputted to the determination model. The determination model is generated in advance based on each of a plurality of learning data sets generated based on the sample shape data, and is further reinforced by learning. Thus, although the shape data is acquired under the conditions different from the inspection conditions for acquiring the sample shape data, the shape of the weld 201 can be accurately evaluated, and whether the shape is good or bad can be correctly determined.

In the acceleration/deceleration section in which the travel speed V changes, the resolution of the shape data is converted and corrected to the same value as the resolution of the sample shape data, and part of the inspection section for measuring the shape data is set as the acceleration section. This allows appearance inspection of the weld 201 to be started from the state in which the shape measurement unit 21 is stationary without providing the approach section separately from the inspection section. Without the need of the approach section, the inspection can be performed in a short time, reducing the production takt time including the appearance inspection.

Also in the acceleration section in which the resolution changes with time, correcting the resolution of the shape data by the conversion/correction described above data can reduce erroneous recognition of the shape defect by the determination model when the shape data is inputted to the determination model. Thus, in the inspection section including the acceleration section, the shape of the weld 201 can be accurately evaluated, and whether the shape is good or bad can be correctly determined.

[Example 5]

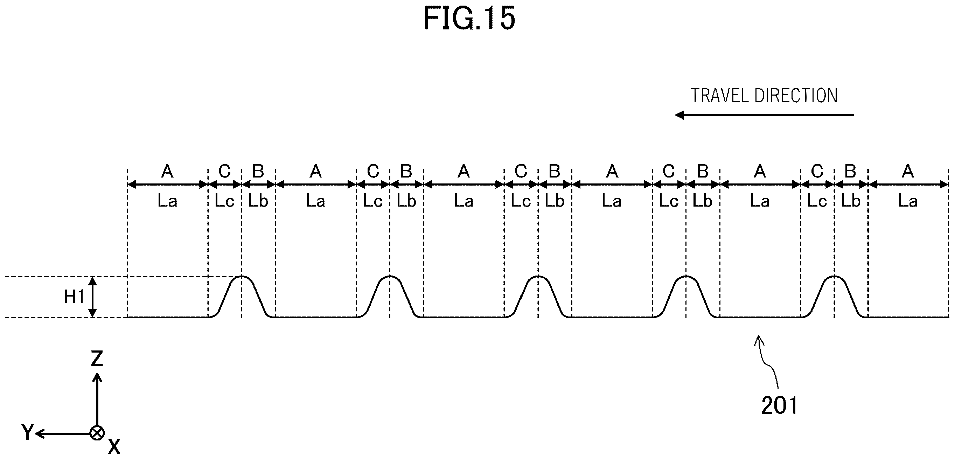

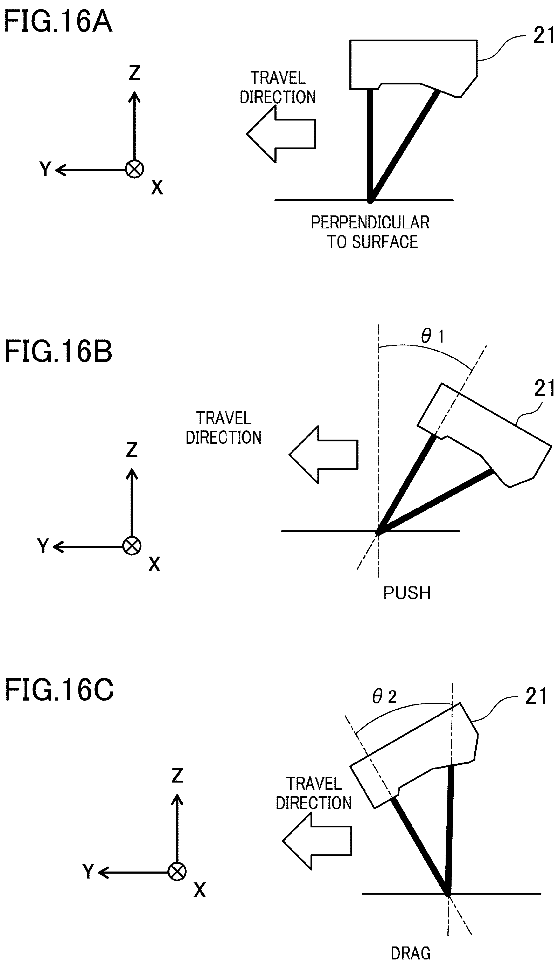

FIG. 15 is a schematic view of a Z-direction profile of a weld according to Example 5. FIG. 16A is a schematic view of arrangement of the shape measurement unit in a section A. FIG. 16B is a schematic view of arrangement of the shape measurement unit in a section B. FIG. 16C is a schematic view of arrangement of the shape measurement unit in a section C. The weld 201 shown in FIG. 15 has an inspection section including the section A which is flat, the section B which is inclined forward in the travel direction, and the section C which is inclined rearward in the travel direction. The sections A, B, and C are periodically arranged in the travel direction.

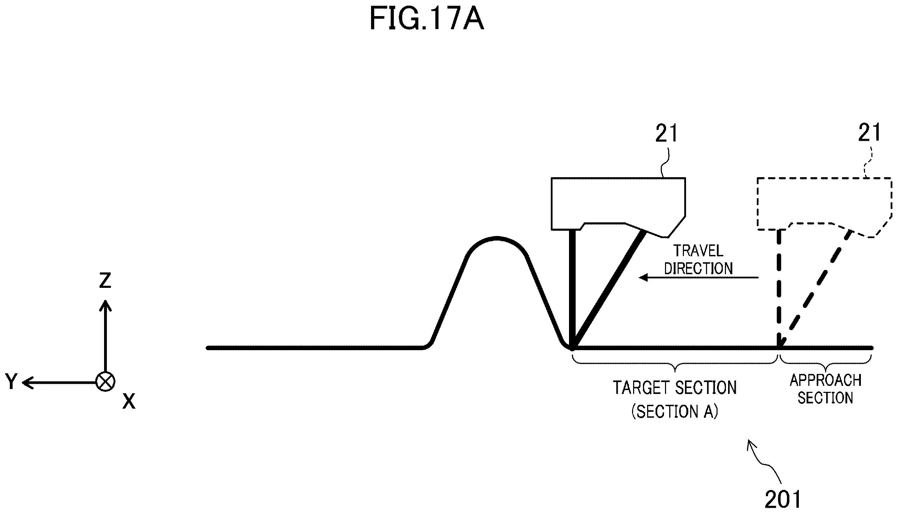

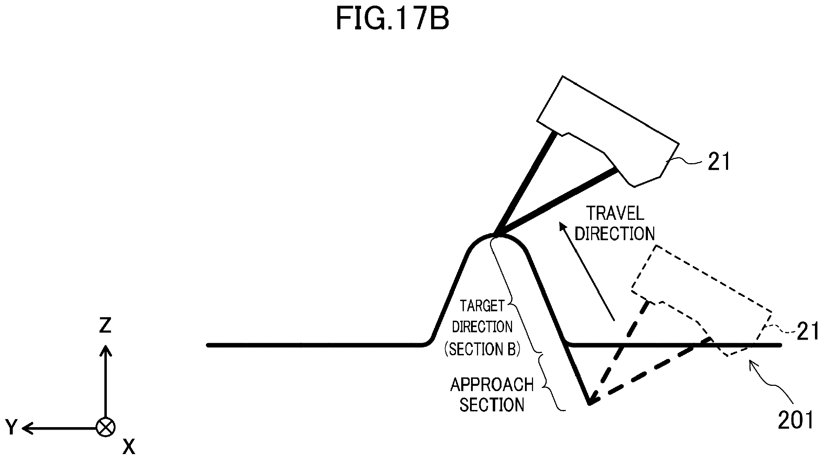

FIG. 17A is a schematic view of arrangement of the shape measurement by a conventional method for the inspection of the section A. FIG. 17B is a schematic view of arrangement of the shape measurement unit by a conventional method for the inspection of the section B.

The weld 201 has various shapes and includes, for example, a weld 201 in which bumps are periodically protruding in the Z direction as shown in FIG. 15.

For the appearance inspection of the weld 201 shown in FIG. 15, the inspection section includes the flat section A, the section B which is inclined forward in the travel direction of the robot 16, and the section C which is inclined rearward in the travel direction of the robot 16. The sections A, B, and C are periodically arranged in this order in the travel direction. The bumps have a height H1 in the Z direction with reference to the surface of the workpiece 200.

When the three-dimensional shape measurement sensor described above is used as the shape measurement unit 21, the appearance inspection of the weld 201 is performed with a work distance, which is a distance between the light receiving surface of the camera or the light receiving sensor array 21b and the surface of the weld 201, kept constant. This is because the shape cannot be measured correctly if the work distance changes. Thus, when the height or inclination of the surface of the weld 201 greatly changes in part of the inspection section, the shape measurement unit 21 needs to be arranged differently before and after the shape measurement unit 21 passes the part to maintain the work distance. Specifically, it is necessary to change the angle of the light receiving surface of the shape measurement unit 21 with respect to the travel direction.

For the appearance inspection of the weld 201 shown in FIG. 15, the shape evaluation unit 21 is arranged so that the optical axis of the laser beam received by the shape evaluation unit 21 is perpendicular to the surface of the weld 201, the surface of the workpiece 200 in this case, in the section A as shown in FIG. 16A. In the section B, as shown in FIG. 16B, the shape evaluation unit 21 is arranged to form a push angle $\theta 1$ in accordance with the inclination of the bump. The push angle $\theta 1$ is an angle at which the shape evaluation unit 21 is inclined opposite to the travel direction with respect to the direction perpendicular to the surface of the workpiece 200 in the flat section A so that the shape evaluation unit 21 receives the laser beam having the optical axis perpendicular to the inclined surface of the section B which is inclined forward in the travel direction. In the section C, as shown in FIG. 16C, the shape evaluation unit 21 is arranged to form a drag angle $\theta 2$ in accordance with the inclination of the bump. The drag angle $\theta 1$ is an angle at which the shape evaluation unit 21 is inclined toward the travel direction with respect to the direction perpendicular to the surface of the workpiece 200 in the flat section A so that the shape evaluation unit 21 receives the laser beam having the optical axis perpendicular to the inclined surface of the section C which is inclined rearward in the travel direction.

When the appearance inspection is continuously performed while changing the arrangement of the shape evaluation unit 21, the work distance also changes before and after passing the part having different inclination or height, and thus the shape of the weld 201 cannot be correctly evaluated.

Thus, in the conventional method, the shape measurement unit 21 is temporarily stopped before and after passing the part in which the height or inclination of the surface of the weld 201 greatly changes. After the arrangement is changed, the shape measurement unit 21 is moved again to restart the shape measurement of the weld 201. In this case, as described in Example 4, the conventional method requires an approach section provided separately from the inspection section in which the shape data is measured. For example, as shown in FIG. 17A, the approach section of a predetermined length is required behind (in the direction opposite to the travel direction) the start position of a measurement target section (the flat section A described above) which is the inspection section. As shown in FIG. 17B, the approach section of a predetermined length is also required behind (in the direction opposite to the travel direction) the start position of the measurement target section (the section B which is inclined forward in the travel direction) which is the inspection section. In the example shown in FIG. 17B, the approach section also includes a component along the Z direction, and the shape measurement unit 21 needs to be move also in the Z direction.

However, as described above, the approach section provided for the shape measurement of the weld 201 disadvantageously increases the production takt time. In particular, as shown in FIG. 15, when the weld 201 includes two or more parts where the height or inclination of the surface greatly changes, the production takt time increases more remarkably.

According to the method disclosed in the present specification, as described above, the shape of the weld 201 can be evaluated without providing the approach section separately from the inspection section. This can significantly keep the production takt time from increasing. This will be described below by way of the example.

For the measurement of the weld 201 shown in FIG. 15, scanning distances La to Lc of the shape measurement unit 21 in the sections A to C are substantially the same value, and are 67.5 mm. The scanning distance in this case is a travel distance of the shape measurement unit 21 along the surface of the weld 201.

It was found from a preliminary experiment that 0.4 sec is required to accelerate the shape measurement unit 21 from a stationary state to a constant speed of 9 m/min, and the travel distance of the shape measurement unit 21 during the acceleration is 30 mm. Thus, in each of the sections A to C, the scanning distance in the acceleration section is 30 mm, and the scanning distance in the constant speed section is 37.5 mm.

As is clear from FIG. 15, the weld 201 includes six sections A, five sections B, and five sections C. Thus, in the sections (6 + 5 + 5 = 16 sections) for the shape measurement, the total of the scanning distances in the acceleration section is 480 mm obtained by 30 (mm) × 16 (sections), and the total of the scanning distances in the constant speed section is 600 mm obtained by 37.5 (mm) × 16 (sections).

In the scanning time taken by the shape measurement unit 21 to scan the weld 201 (corresponding to the travel time of the robot 16), time taken to scan the acceleration section is 6.4 (sec) obtained by 16 (sections) × 0.4 (sec). Time taken to scan the constant speed section is 4.0 (sec) obtained by 16 (sections) × 37.5 (sec) / 9 (m/min) = 16 (sections) × 37.5 (sec) / 150 (mm/sec). Table 1 collectively shows the results.

[Table 1]

| | | ACCELERATION SECTION | CONSTANT SPEED SECTION | TOTAL SECTION (ACCELERATION SECTIONS + CONSTANT SPEED SECTIONS) |
|---|---|---|---|---|
| SCANNING DISTANCE [mm] | PER SECTION | 30 | 37.5 | 67.5 |
| | ALL SECTIONS IN TOTAL | 480 | 600 | 1080 |
| SCANNING TIME [sec] | PER SECTION | 0.4 | 0.25 | 0.65 |
| | ALL SECTIONS IN TOTAL | 6.4 | 4.0 | 10.4 |

The appearance inspection of the weld 201 was performed in the same manner as in Example 4. That is, in each constant speed section, the original shape data was reconstructed using the X-direction resolutions $Rx$ and $Rx_0$ and the Y-direction resolutions $Ry$ and $Ry_0$, and the values of the Z coordinates as the coordinates after the correction were corrected based on Formula (4) for each point of the reconstructed point group data. Thus, the resolution of the shape data was corrected. The first determination unit 28 determined whether the shape of the weld 201 was good or bad in each of the constant speed sections using the learned determination model that was generated based on each of the plurality of learning data sets and further reinforced by learning. As described above, each of the learning data sets was generated based on the sample shape data acquired in advance.

In each acceleration section, the original shape data was reconstructed using the X-direction resolutions $Rx$ and $Rx_0$, the Y-direction resolutions $Ry(t)$ and $Ry_0$, and the resolution coefficient $Cy_m(t)$, and Z ($Xn/Cx$, $Ym/Cy_m(t)$) as

the coordinates after the correction shown in Formula (7) was calculated for all the point groups included in the acceleration section. Further, (Xn/Cx, Ym/Cy$_m$(t), Z (Xn/Cx, Ym/Cy$_m$(t))) was calculated for all the coordinate points included in the acceleration section, and the shape data with corrected resolution was obtained in each acceleration section. The first determination unit 28 determined whether the shape of the weld 201 was good or bad in each acceleration section using the learned determination model.

As a comparative example, the shape of the weld 201 was measured by a conventional method. In the comparative example, as described above, the approach section was provided separately from the inspection section in which the shape data was measured for the shape measurement in each of the sections A to C. In the comparative example, the shape measurement unit 21 was moved backward from the end point of the measurement of the section A by the distance of the approach section. The angle of the light receiving surface of the shape measurement unit 21 with respect to the surface of the weld 201 was changed so that the optical axis of the laser beam received by the shape evaluation unit 21 was perpendicular to the surface of the workpiece 200. Thereafter, the shape measurement unit 21 was moved at an accelerating speed to the measurement start point. Further, the shape of the weld 201 was measured while moving the shape measurement unit 21 at a constant speed from the measurement start point to measurement end point of the section A. These steps were performed in each of the six sections A which were flat, the five sections B which were inclined forward in the travel direction, and the five sections C which were inclined backward in the travel direction to acquire the shape data of the weld 201.

The sample shape data was acquired with the travel speed V of the robot 16 fixed to Vc (= 9 m/min), and a determination model that was reinforced by learning based on the sample shape data was prepared in advance. The shape of the weld 201 was evaluated using this determination model. The first determination unit 28 determined whether the shape of the weld 201 was good or bad in each of the sections A to C using the learned determination model, and the determination results of the whole inspection section were collected to output the final determination result.

In the comparative example, the scanning distance and scanning time of the shape measurement unit 21 in each approach section provided separately from the inspection section for the shape data measurement were added to the scanning distance and scanning time of the constant speed section to calculate the total section (approach sections + constant speed sections) as the entire scanning distance and scanning time. Table 2 collectively shows the results of this example in comparison with the results of the present example. The shape measurement unit 21 travels the approach section from and to the measurement start point of each inspection section. Thus, the scanning distance and scanning time of the shape measurement unit 21 in the approach section were respectively twice the values of the present example. Specifically, the scanning distance of the approach section was 30 (mm) × 2 = 60 (mm) per section, and the scanning time was 0.4 (sec) × 2 = 0.8 (sec) per section.

[Table 2]

| | | APPROACH SECTION (INCLUDING TRAVEL TO START POINT OF APPROACH SECTION) | CONSTANT SPEED SECTION | TOTAL SECTION (ACCELERATION SECTIONS + CONSTANT SPEED SECTIONS) | TOTAL SECTION/ TOTAL SECTION BY CONVENTIONAL METHOD |
|---|---|---|---|---|---|
| SCANNING DISTANCE [mm] | PER SECTION | 60 | 37.5 | 97.5 | 69.2% (=> REDUCTION BY 30.8%) |
| | ALL SECTIONS IN TOTAL | 960 | 600 | 1560 | 69.2% (=> REDUCTION BY 30.8%) |
| SCANNING TIME [see] | PER SECTION | 0.8 | 0.45 | 1.25 | 52% (=> REDUCTION BY 48%) |
| | ALL SECTIONS IN TOTAL | 12.8 | 7.2 | 20 | 52% (=> REDUCTION BY 48%) |

As is clear from Table 2, in the present example, the scanning distance of the shape measurement unit 21 was reduced by about 30% and the scanning time was reduced by about 50%, compared to those of the comparative example.

That is, an increase in the production takt time required for the appearance inspection was significantly reduced.

Further, correcting the resolution of the shape data measured by the shape measurement unit 21 to the same value as the resolution of the sample shape data acquired in advance can reduce erroneous recognition of the shape defect by the determination model when the shape data is inputted to the determination model. Thus, although the shape data is acquired under the conditions different from the inspection conditions for acquiring the sample shape data, the shape of the weld 201 can be accurately evaluated, and whether the shape is good or bad can be correctly determined.

As shown in FIG. 15, although the height and inclination of the weld 201 greatly change, the appearance of the weld 201 can be inspected without providing an approach section separately from the inspection section by dividing the inspection section for the measurement of the shape data into the acceleration section and the constant speed section.

Also in the acceleration section in which the resolution changes with time, the resolution of the shape data is converted/corrected as described above so that the resolution of the shape data has the same value as the resolution of the sample shape data acquired in advance. This can reduce erroneous recognition of the shape defect by the determination model that is generated based on each of the learning data sets and further reinforced by learning when the shape data is inputted to the determination model. Each of the learning data sets is generated in advance based on the sample shape data. Thus, in the inspection section including the acceleration section, the shape of the weld 201 can be accurately evaluated, and whether the shape is good or bad can be correctly determined.

(Other Embodiments)

In the example shown in FIG. 1, both of the welding torch 11 (welding head 11) and the shape measurement unit 21 are attached to the robot 16. However, the shape measurement unit 21 may be attached to a robot (not shown) different from the robot 16 to which the welding torch 11 (welding head 11) is attached. In this case, various types of data are transmitted to the data processor 23 from another robot controller (not shown) configured to control the motion of the different robot.

The learning data set generator 26 according to the embodiment classifies the sample shape data by material and shape of the workpiece 200 and performs data augmentation on the classified pieces of sample shape data to generate the learning data sets.

However, the learning data set generator 26 may not have the classifying function. In this case, the determination model generator 27 may not have the function of generating the determination model for each material and shape of the workpiece 200.

INDUSTRIAL APPLICABILITY

The appearance inspection apparatus of the present disclosure can accurately evaluate the three-dimensional shape of welds although inspection conditions are changed, and thus is particularly useful for appearance inspection of a workpiece including various types of welds.

DESCRIPTION OF REFERENCE CHARACTERS

| 10 | Welding Apparatus |
| 11 | Welding Head (Welding Torch) |
| 12 | Welding Wire |
| 13 | Wire Feeder |
| 14 | Power Supply |
| 15 | Output Controller |
| 16 | Robot |
| 17 | Robot Controller |
| 20 | Appearance Inspection Apparatus |
| 21 | Shape Measurement Unit |
| 22 | Sensor Controller |
| 23 | Data Processor |
| 24 | Shape Data Processor |
| 25 | First Storage |
| 26 | Learning Data Set Generator |
| 27 | Determination Model Generator |
| 28 | First Determination Unit |

29     Notification Unit
100    Welding System
200    Workpiece
201    Weld

**Claims**

1. An appearance inspection apparatus for inspecting an appearance of a weld of a workpiece, the appearance inspection apparatus comprising at least:

   a shape measurement unit that is attached to a robot and configured to measure a three-dimensional shape of the weld along a welding line; and
   a data processor configured to process shape data acquired by the shape measurement unit,
   the data processor including at least:

   a shape data processor configured to perform at least correction of a resolution of the shape data acquired by the shape measurement unit;
   a learning data set generator configured to generate a plurality of learning data sets by performing data augmentation on multiple pieces of sample shape data acquired in advance by the shape measurement unit;
   a determination model generator configured to generate a determination model for determining whether the shape of the weld is good or bad using the plurality of learning data sets; and
   a first determination unit configured to determine whether the shape of the weld is good or bad based on the shape data corrected by the shape data processor and one or more determination models generated by the determination model generator.

2. The appearance inspection apparatus of claim 1, wherein
   the shape data processor corrects the resolution of the shape data acquired by the shape measurement unit based on a measurement resolution, measurement frequency, and scanning speed of the shape measurement unit.

3. The appearance inspection apparatus of claim 2, wherein

   the sample shape data is acquired at the measurement resolution, the measurement frequency, and the scanning speed that are determined in advance, and
   the shape data processor corrects the resolution of the shape data acquired by the shape measurement unit to the same value as a resolution of the sample shape data.

4. The appearance inspection apparatus of claim 2 or 3, further comprising:

   a sensor controller configured to store a condition for inspection by the shape measurement unit and transmit the stored inspection condition to the data processor, wherein
   the sensor controller transmits the measurement resolution in an X direction intersecting with a Y direction along the welding line and a Z direction which is a height direction of the weld and the measurement frequency to the data processor, and
   the data processor receives a travel speed or speed control function of the robot from a robot controller configured to control a motion of the robot.

5. The appearance inspection apparatus of claim 4, wherein

   the shape data processor corrects a value of the shape data in the Z direction based on an X-direction resolution and Y-direction resolution of the shape data,
   the Y-direction resolution is determined based on the measurement frequency and the travel speed of the robot when the shape measurement unit measures the three-dimensional shape of the weld while the robot travels along the welding line at a constant speed,
   the Y-direction resolution is determined based on the measurement frequency and the speed control function of the robot when the shape measurement unit measures the three-dimensional shape of the weld while the robot travels at an accelerating speed, a decelerating speed, or both accelerating and decelerating speeds in a predetermined section along the welding line, and

the speed control function of the robot is a function dependent on time.

6. The appearance inspection apparatus of any one of claims 1 to 5, wherein

the learning data set generator classifies the multiple pieces of sample shape data acquired by the shape measurement unit by material and shape of the workpiece and performs data augmentation on the classified pieces of sample shape data to generate the plurality of learning data sets, and
the determination model generator generates the determination model for each material and shape of the workpiece using the plurality of learning data sets.

7. The appearance inspection apparatus of any one of claims 1 to 6, wherein

the data processor further includes a first storage configured to store at least the sample shape data, and
the learning data set generator configured to read the sample shape data stored in the first storage to generate the plurality of learning data sets.

8. The appearance inspection apparatus of any one of claims 1 to 7, wherein
the data processor further includes a notification unit configured to notify a result of the determination by the first determination unit.

9. The appearance inspection apparatus of any one of claims 1 to 8, wherein

the determination model is reinforced by learning using the learning data set,
the learning data set includes:

non-defective data which is shape data including no shape defect in the weld; and
learning data obtained by identifying, in defective data which is shape data including a shape defect in the weld, a type of the shape defect and labelling the shape defect with the type,

the first determination unit inputs the shape data inputted from the shape data processor to the determination model, and
the determination model determines whether the shape defect is present and identifies a type, number, and size of the shape defect and a location of the shape defect with respect to the weld to determine whether the shape of the weld is good or bad based on the results of the determination and the identification.

10. A welding system, comprising:

the appearance inspection apparatus of any one of claims 1 to 9; and
a welding apparatus configured to weld the workpiece, wherein
the welding apparatus includes at least:

a welding head configured to apply heat to the workpiece; and
an output controller configured to control a welding output of the welding head.

11. The welding system of claim 10, wherein

the welding apparatus includes at least:

the robot configured to hold the welding head and moves the welding head to a desired position; and
a robot controller configured to control a motion of the robot, and

when the first determination unit determines that the shape of the weld is bad, the output controller stops the welding output of the welding head, and the robot controller stops the motion of the robot or operates the robot so that the welding head moves to a predetermined initial position.

12. A method for correcting shape data acquired by the appearance inspection apparatus of any one of claims 1 to 9, the method comprising:

measuring a three-dimensional shape of the weld by the shape measurement unit moving together with the robot to acquire the sample shape data for generating the plurality of learning data sets;

measuring the three-dimensional shape of the weld by the shape measurement unit moving together with the robot to acquire the shape data; and

correcting, when a resolution of the shape data acquired by the shape measurement unit is different from a resolution of the sample shape data, the shape data by the shape data processor so that the resolution of the shape data acquired by the shape measurement unit has the same value as the resolution of the sample shape data.

**13.** The method of claim 12, wherein

the shape data processor corrects a value of the shape data in a Z direction which is a height direction of the weld based on an X-direction resolution and Y-direction resolution of the shape data,

the Y-direction resolution is determined based on a travel speed of the robot and a measurement frequency of the shape measurement unit when the shape measurement unit measures the three-dimensional shape of the weld while the robot travels along the welding line at a constant speed,

the Y-direction resolution is determined based on the measurement frequency and the speed control function of the robot when the shape measurement unit measures the three-dimensional shape of the weld while the robot travels at an accelerating speed, a decelerating speed, or both accelerating and decelerating speeds in a predetermined section along the welding line, and

the speed control function of the robot is a function dependent on time.

**14.** A method for appearance inspection of a weld using the appearance inspection apparatus of any one of claims 1 to 9, the method comprising:

measuring a three-dimensional shape of the weld by the shape measurement unit moving together with the robot to acquire the sample shape data for generating the plurality of learning data sets;

generating one or more determination models for determining whether the shape of the weld is good or bad by the determination model generator using the plurality of learning data sets;

measuring the three-dimensional shape of the weld by the shape measurement unit moving together with the robot to acquire the shape data;

correcting, when a resolution of the shape data acquired by the shape measurement unit is different from a resolution of the sample shape data, the shape data by the shape data processor so that the resolution of the shape data acquired by the shape measurement unit has the same value as the resolution of the sample shape data; and

determining whether the shape of the weld is good or bad by the first determination unit based on the shape data having the resolution corrected by the shape data processor and the one or more determination models generated by the determination model generator.

**15.** The method of claim 14, wherein

the shape data processor corrects a value of the shape data in a Z direction which is a height direction of the weld based on an X-direction resolution and Y-direction resolution of the shape data,

the Y-direction resolution is determined based on a travel speed of the robot and a measurement frequency of the shape measurement unit when the shape measurement unit measures the three-dimensional shape of the weld while the robot travels along the welding line at a constant speed,

the Y-direction resolution is determined based on the measurement frequency and the speed control function of the robot when the shape measurement unit measures the three-dimensional shape of the weld while the robot travels at an accelerating speed, a decelerating speed, or both accelerating and decelerating speeds in a predetermined section along the welding line, and

the speed control function of the robot is a function dependent on time.

**16.** The method of claim 14 or 15, wherein

the determination model is reinforced by learning using the learning data set,
the learning data set includes:

non-defective data which is shape data including no shape defect in the weld; and

learning data obtained by identifying, in defective data which is shape data including a shape defect in the weld, a type of the shape defect and labelling the shape defect with the type,

the determining whether the shape of the weld is good or bad by the first determination unit includes:

determining whether the shape defect is present based on the shape data inputted by the shape data processor and the determination model;

identifying a number and size of the shape defect and a location of the shape defect with respect to the weld; and

identifying a type of the shape defect, and

the first determination unit determines whether the shape of the weld is good or bad based on the results of the determination and the identification of each of the sub-steps.

# FIG.1

# FIG.2

# FIG.3

EP 4 455 645 A1

# FIG.4

# FIG.5A

22

22a

SHAPE
MEASUREMENT UNIT

DATA PROCESSOR

CPU

RAM

22b

# FIG.5B

23

SHAPE CONTROLLER →
SENSOR CONTROLLER →

23e INPUT PORT

23d IC

23a CPU

23b GPU

23c RAM/ROM

23f OUTPUT PORT

→ ROBOT CONTROLLER
→ OUTPUT CONTROLLER

23h

23g DISPLAY

EP 4 455 645 A1

# FIG.6A

# FIG.6B

201

ΔH

H

200

Z

Y ⊗ → X

# FIG.6C

202

200

Z

Y ⊗ → X

# FIG.6D

# FIG.6E

# FIG.7A

ORIGINAL SHAPE DATA

DATA AUGMENTATION ⇒ LEARNING DATA SET

# FIG.7B

ORIGINAL SHAPE DATA

DATA AUGMENTATION ⇒ LEARNING DATA SET

# FIG.7C

ORIGINAL SHAPE DATA

DATA AUGMENTATION ⇒ LEARNING DATA SET

# FIG.8A

START

MEASURE SHAPE OF WELD BEAD — S1

DIVIDE SHAPE DATA INTO CONSTANT SPEED SECTIONS OR ACCELERATION/ DECELERATION SECTIONS — S2

EXECUTE PROCESSES SUCH AS NOISE REMOVAL ON SHAPE DATA OF SELECTED DIVIDED SECTION — S3

S4 — SPEED CONTROL FUNCTION OF ROBOT IN SELECTED DIVIDED SECTION SHOWS CONSTANT SPEED?

NO →

STORE X-DIRECTION RESOLUTION — S10

CALCULATE Y-DIRECTION RESOLUTION BASED ON SPEED CONTROL FUNCTION OF ROBOT — S11

CORRECT RESOLUTION OF SHAPE DATA IN ACCELERATION/ DECELERATION SECTION — S12

DETERMINE WHETHER THE SHAPE OF WELD BEAD IS GOOD OR BAD USING LEARNED DETERMINATION MODEL — S13

YES

S5 — CALCULATE AND STORE X-DIRECTION/Y-DIRECTION RESOLUTIONS

S6 — SHAPE DATA HAS THE SAME X-DIRECTION/Y-DIRECTION RESOLUTIONS AS STORED SAMPLE SHAPE DATA?

YES →

NO

S7 — CORRECT RESOLUTION OF SHAPE DATA IN CONSTANT SPEED SECTION

DETERMINE WHETHER THE SHAPE OF WELD BEAD IS GOOD OR BAD USING LEARNED DETERMINATION MODEL — S8

S9 — ANY SECTION WITH PROCESSES SUCH AS NOISE REMOVAL UNDONE ON SHAPE DATA LEFT?

YES

NO

END

# FIG.8B

STEP S7
(STEP S12)

SA — DETERMINE WHETHER SHAPE DEFECT IS PRESENT

SB — IDENTIFY SIZE, NUMBER AND LOCATION OF SHAPE DEFECT

SC — IDENTIFY TYPE OF SHAPE DEFECT

S8
(S13)

STEP S9

# FIG.9

Z

X

Y

Z(x, y)  dx  1−dx  Z(x+Rx, y)

dy

COORDINATES AFTER CORRECTION

Z(Xn/Cx, Ym/Cy)

1−dy

Z(x, y+Ry)  Z(x+Rx, y+Ry)

## FIG.10

BEFORE CORRECTION

Y-DIRECTION RESOLUTION CHANGES WITH TRAVEL SPEED OF ROBOT

X-DIRECTION RESOLUTION

Y-DIRECTION RESOLUTION

X-DIRECTION

Y-DIRECTION

AFTER CORRECTION

X-DIRECTION RESOLUTION

Y-DIRECTION RESOLUTION

X-DIRECTION

Y-DIRECTION

# FIG.11

# FIG.12

TRAVEL DIRECTION →

21

201

Z
↑
X ⊙ → Y

FIG.13

SECTION I  SECTION II  SECTION III  SECTION IV  SECTION V

TRAVEL DIRECTION

TRAVEL DIRECTION

201

FIG.14

TRAVEL
DIRECTION

21

201

ACCELERATION SECTION

(APPROACH SECTION)

CONSTANT SPEED SECTION

Z

X ⊙ → Y

EP 4 455 645 A1

# FIG.15

EP 4 455 645 A1

TRAVEL DIRECTION

| A | C | B | A | C | B | A | C | B | A | C | B | A | C | B | A |
| La | Lc | Lb | La | Lc | Lb | La | Lc | Lb | La | Lc | Lb | La | Lc | Lb | La |

H1

201

Z

Y ⊗ X

## FIG.16A

TRAVEL
DIRECTION

21

Z
Y ← ⊗ X

PERPENDICULAR
TO SURFACE

## FIG.16B

$\theta 1$

21

TRAVEL
DIRECTION

Z
Y ← ⊗ X

PUSH

## FIG.16C

$\theta 2$

21

TRAVEL
DIRECTION

Z
Y ← ⊗ X

DRAG

# FIG.17A

# FIG.17B

21

TRAVEL
DIRECTION

TARGET
DIRECTION
(SECTION B)

APPROACH
SECTION

21

201

Z

Y ⊗ X

## INTERNATIONAL SEARCH REPORT

| International application No. |
|---|
| **PCT/JP2022/044531** |

**A. CLASSIFICATION OF SUBJECT MATTER**

*G01N 21/88*(2006.01)i; *B23K 31/00*(2006.01)i; *G01B 11/24*(2006.01)i; *G06N 20/00*(2019.01)i
FI: G01N21/88 Z; B23K31/00 Z; B23K31/00 K; G06N20/00; G01B11/24 K

According to International Patent Classification (IPC) or to both national classification and IPC

**B. FIELDS SEARCHED**

Minimum documentation searched (classification system followed by classification symbols)
G01N21/84-G01N21/956; B23K31/00-B23K31/12; G01B11/00-G01B11/30; G06N20/00-20/20

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2020/129617 A1 (PANASONIC INTELLECTUAL PROPERTY MANAGEMENT CO., LTD.) 25 June 2020 (2020-06-25) entire text, all drawings | 1-16 |
| A | WO 2019/065578 A1 (FUJIFILM CORP.) 04 April 2019 (2019-04-04) entire text, all drawings | 1-16 |
| A | JP 2018-205024 A (KEYENCE CORP.) 27 December 2018 (2018-12-27) entire text, all drawings | 1-16 |
| A | JP 2015-094675 A (RICOH CO., LTD.) 18 May 2015 (2015-05-18) entire text, all drawings | 1-16 |
| A | JP 8-327559 A (NEC CORP.) 13 December 1996 (1996-12-13) entire text, all drawings | 1-16 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **09 February 2023** | **21 February 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2022/044531**

| Patent document cited in search report | | | Publication date (day/month/year) | Patent family member(s) | | | Publication date (day/month/year) |
|---|---|---|---|---|---|---|---|
| WO | 2020/129617 | A1 | 25 June 2020 | EP 3900870 A1 entire text, all drawings CN 113207286 A | | | |
| WO | 2019/065578 | A1 | 04 April 2019 | (Family: none) | | | |
| JP | 2018-205024 | A | 27 December 2018 | (Family: none) | | | |
| JP | 2015-094675 | A | 18 May 2015 | (Family: none) | | | |
| JP | 8-327559 | A | 13 December 1996 | US 5784484 A entire text, all drawings | | | |

Form PCT/ISA/210 (patent family annex) (January 2015)

**EP 4 455 645 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020129617 A